(19) Europäisches Patentamt
European Patent Office
Office européen des brevets

(11) Numéro de publication: **0 053 074 B1**

(12) # FASCICULE DE BREVET EUROPEEN

(45) Date de publication du fascicule du brevet: **19.09.84**

(51) Int. Cl.$^3$: **C 07 D 501/24**

(21) Numéro de dépôt: **81401832.1**

(22) Date de dépôt: **19.11.81**

(54) **Nouveaux dérivés de la céphalosporine et leur préparation.**

(30) Priorité: **20.11.80 FR 8024638**

(43) Date de publication de la demande: **02.06.82 Bulletin 82/22**

(45) Mention de la délivrance du brevet: **19.09.84 Bulletin 84/38**

(84) Etats contractants désignés: **AT BE CH DE FR GB IT LI LU NL SE**

(56) Documents cités:
**EP-A-0 053 542**
**FR-A-2 081 451**
**FR-A-2 137 899**

(73) Titulaire: **RHONE-POULENC SANTE**
**Les Miroirs 18 Avenue d'Alsace**
**F-92400 Courbevoie Cedex (FR)**

(72) Inventeur: **Berger, Christian**
**26 A rue Paul Rivet**
**F-92350 Le Plessis-Robinson (FR)**
Inventeur: **Farge, Daniel**
**30 rue des Pins Sylvestres**
**F-94320 Thiais (FR)**
Inventeur: **Moutonnier, Claude**
**3 rue Auguste Rodin**
**F-92350 Le Plessis-Robinson (FR)**
Inventeur: **Wolff, Gérard**
**7 rue Jeanne d'Arc**
**F-94320 Thiais (FR)**

(74) Mandataire: **Gaumont, Robert et al**
**RHONE-POULENC RECHERCHES Service**
**Brevets Pharma 25, Quai Paul Doumer**
**F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 053 074 B1

**Description**

La présente invention concerne de nouveaux dérivés de vinyl-3 céphalosporines de formule générale:

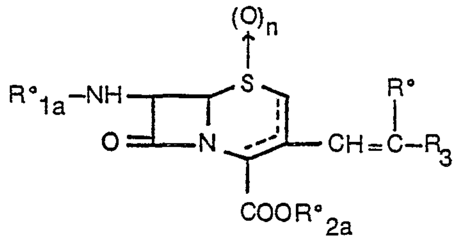

(I)

et leur préparation.

Dans FR—A—2 137 899 ont été décrites des céphalosporines de formule générale:

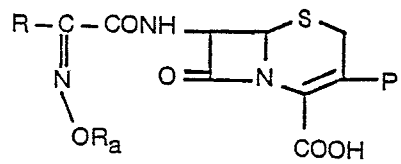

dans laquelle R peut être isothiazolyle et P représente un groupe organique, notamment alcoxy-carbonylvinyle substitué.

Dans FR—A—2 081 451 ont été décrites des céphalosporines représentées par la formule générale:

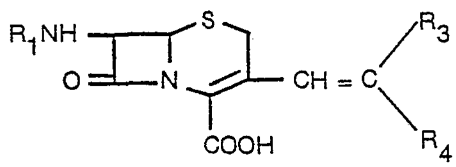

dans laquelle $R_1$ représente divers radicaux acyle et $R_3$ et $R_4$ représentent des atomes d'hydrogène ou des radicaux aliphatiques ou aromatiques substitués ou non.

Les produits de formule générale (I), dans laquelle n égale 0 ou 1, se présentent sous forme bicyclooctène-2 et/ou -3 lorsque n = 0 (selon la nomenclature des Chemical Abstracts) et sous forme bicyclooctène-2 lorsque n = 1; le symbole $R°_{1a}$ représente

a) un radical de formule générale:

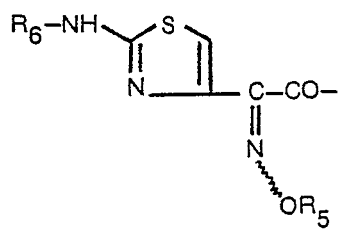

(II)

[dans laquelle $R_5$ est un atome d'hydrogène, un radical alcoyle, vinyle, cyanométhyle, un groupement protecteur d'oxime tel que trityle, tétrahydropyranyle ou méthoxy-2 propyle-2 ou un radical de formule générale:

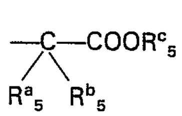

(III)

(dans laquelle $R^a_5$ et $R^b_5$, qui sont identiques ou différents, sont des atomes d'hydrogène, des radicaux alcoyle ou forment ensemble un radical alcoylène contenant 2 ou 3 atomes de carbone et $R^c_5$ est un atome d'hydrogène ou un radical protecteur d'acide, par exemple tel que cité ci-après pour $R°_{2a}$) et $R^6$ est un atome d'hydrogène ou un radical protecteur d'amino tel que t.butoxycarbonyle, trityle, benzyle, dibenzyle, benzyloxycarbonyle, p.nitrobenzyloxycarbonyle, p.méthoxybenzyloxycarbonyle, ou tel qu'un radical (IVc) défini ci-après]

b) un atome d'hydrogène, ou

c) un radical benzhydryle, trityle, un radical acyle de formule générale:

$$R_7—CO— \quad (IVa)$$

[dans laquelle $R_7$ est un radical alcoyle (substitué par un radical phényle ou phénoxy)] un radical de formule générale:

$$R_8O\ CO— \quad (IVb)$$

[dans laquelle $R_8$ est un radical alcoyle ramifié non substitué ou alcoyle ramifié ou droit portant 1 ou plusieurs substituants [choisis parmi phényle et phényle substitué (par un radical alcoyloxy, nitro ou phényle)], ou vinyle] un radical de formule générale:

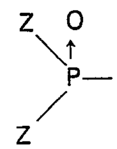

(IVc)

dans laquelle les symboles Z représentent des radicaux phényle ou —OZ' dans lequel Z' représente un radical alcoyle, trichloro-2,2,2-éthyle, phényle ou benzyle (ces 2 derniers radicaux pouvant être substitués par un atome d'halogène ou un radical alcoyle, alcoyloxy ou nitro), ou bien les symboles Z' des 2 substituants Z forment ensemble un radical alcoylène contenant 2 ou 3 atomes de carbone ou bien $R^{o}_{1a}NH$ est remplacé par un radical méthylèneamino dans lequel le radical méthylène est substitué par un groupement dialcoylamino ou phényle (lui-même éventuellement substitué par un ou plusieurs radicaux méthoxy ou nitro);

le symbole $R^{o}_{2a}$ représente un atome d'hydrogène ou un radical protecteur d'acide tel que méthoxyméthyle, t.butyle, benzhydryle, benzyle, nitrobenzyle ou p.méthoxybenzyle;

le symbole $R^{o}$ est un radical d'un premier groupe constitué par un radical phényle éventuellement substitué (par un radical alcoyle, trifluorométhyle, dialcoylaminométhyle, alcoyloxy, alcoylthio, dialcoylamino ou par un atome d'halogène tel que le fluor ou le chlore), un radical hétérocyclyle à 5 ou 6 chaînons contenant un seul hétéroatome (tel que pyridyle-2 ou -3, thiényle-2 ou -3 ou furyle-2 ou -3) éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons, ou un radical hétérocyclyle à 5 ou 6 chaînons contenant un atome d'oxygène ou de soufre (dans ce qui suit, un radical du second groupe sera désigné comme "radical —CH $R_9R_{10}$"); et

le symbole $R_3$ représente un radical de formule générale:

$$R_4\ SO_2O— \quad (V)$$

dans laquelle $R_4$ représente un radical alcoyle, trihalogénométhyle (par exemple trifluorométhyle) ou phényle éventuellement substitué par un atome d'halogène ou par un ou plusieurs radicaux alcoyle ou nitro;

étant entendu que les portions ou radicaux alcoyles cités ci-dessus (ou qui seront cités ci-après) sont (sauf mention spéciale) droits ou ramifiés et contiennent 1 à 4 atomes de carbone.

Dans la formule générale (I) les radicaux $R^{o}$ et $R_3$ peuvent se trouver en position cis ou trans.

Il est également entendu que les mélanges des isomères bicyclooctène-2 et -3 et/ou cis et trans entrent dans le cadre de la présente invention.

Dans ce qui suit la stéréoisomérie trans sera désignée par E et la stéréoisomérie cis sera désignée par Z.

Par ailleurs, il est entendu que le groupement $—OR_5$ du radical de formule générale (II) peut se trouver dans l'une des positions syn ou anti et que ces isomères et leurs mélanges entrent aussi dans le cadre de la présente invention.

La forme syn peut être représentée par la formule

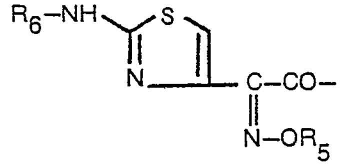

(IIa)

La forme anti peut être représentée par la formule

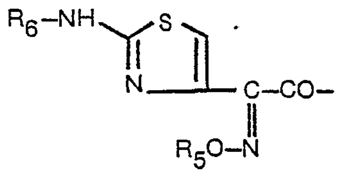

(IIb)

Parmi les significations de $R^{\circ}_{1a}$ définies ci-dessus en (a) et (c) peuvent être citées notamment:
méthoxyimino-2 (tritylamino-2 thiazolyl-4)-2 acétyle
méthoxyimino-2 (t.butoxycarbonylamino-2 thiazolyl-4)-2 acétyle
trityloxyimino-2 (tritylamino-2 thiazolyl-4)-2 acétyle
tétrahydropyranyloxyimino-2 (tritylamino-2 thiazolyl-4)-2 acétyle
t.butoxycarbonylméthoxyimino-2 (tritylamino-2-thiazolyl-4)-2 acétyle,
trityle, phénylacétyle
phénoxyacétyle
t.butoxycarbonyle
benzyloxycarbonyle
p.méthoxybenzyloxycarbonyle
p.nitrobenzyloxycarbonyle
diphénylméthoxycarbonyle
(biphénylyl-4)-2 isopropyloxycarbonyle
vinyloxycarbonyle
diméthoxyphosphoryle,
diéthoxyphosphoryle,
diphénoxyphosphoryle,
dibenzyloxyphosphoryle.

Comme exemples de radicaux méthylèneamino on peut citer:
diméthylaminométhylèneamino
diméthoxy-3,4 benzylidèneamino, nitro-4 benzylidèneamino.

I) Selon l'invention, les produits de formule générale (I) pour lesquels $R^{\circ}_{1a}$, $R^{\circ}_{2a}$, $R_3$, R° et n sont définis comme précédemment, à l'exception pour $R^{\circ}_{1a}$ de représenter un atome d'hydrogène, peuvent être préparés par action d'une forme activée d'un acide $R_4\ SO_3H$ du type:

$$(R_4\ SO_2)_2O \qquad \text{(VIa)}$$

$$\text{ou } R_4\ SO_2\ \text{Hal} \qquad \text{(VIb)}$$

[$R_4$ étant défini comme ci-dessus et Hal étant un atome d'halogène] sur une cétone de formule générale

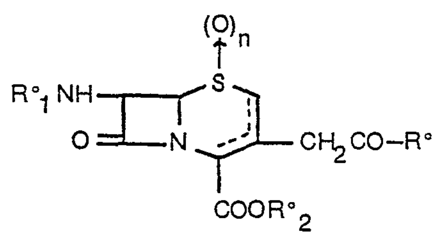

(VII)

[(dans laquelle $R^{\circ}_1$ est défini comme $R^{\circ}_{1a}$ en a) et c), à l'exception pour $R^6$ de représenter un atome d'hydrogène, $R^{\circ}_2$ est défini comme $R^{\circ}_{2a}$, R° et n sont définis comme précédemment, et qui se présente sous forme (oxo-2 éthyl)-3 bicyclooctène-2 ou -3 lorsque n = 0 et sous forme (oxo-2 éthyl)-3 bicyclooctène-2 lorsque n = 1] ou sur un mélange de ses isomères, suivie éventuellement de la réduction du sulfoxyde obtenu lorsque n = 1 et éventuellement de l'élimination des radicaux protecteurs de la fonction amine du radical de formule générale (II) et/ou des fonctions acides.

Il est entendu que, lorsque $R^{\circ}_{1a}$ est un radical de formule générale (II) dans laquelle $R_5$ est un atome d'hydrogène, il est nécessaire que l'oxime soit protégée. La protection et l'élimination s'effectuent selon les méthodes décrites ci-après.

On opère généralement en présence
d'une base tertiaire de formule générale:

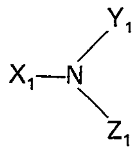

(VIIIa)

dans laquelle $X_1$, $Y_1$ et $Z_1$ représentent des radicaux alcoyle ou phényle, ou éventuellement deux d'entre eux forment un cycle avec l'atome d'azote auquel ils sont rattachés (par exemple la triéthylamine ou la diméthylaniline)

ou d'une pyridine substituée par des radicaux alcoyle ou dialcoylamino (par exemple la di-t.butyl-2,6 méthyl-4-pyridine)

ou d'un amidure de lithium obtenu par réaction d'un alcoyllithium (par exemple le n.butyllithium) sur une amine secondaire de formule générale:

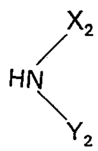

(VIIIb)

dans laquelle $X_2$ et $Y_2$ représentent des radicaux alcoyle ou phényle, ou éventuellement $X_2$ et $Y_2$ forment un cycle avec l'atome d'azote auquel ils sont rattachés (par exemple la diisopropylamine ou la tétraméthyl-2,2,6,6 pipéridine)

ou d'un alcoolate de métal alcalin (par exemple le t.butylate de potassium)

ou d'un hydrure de métal alcalin (par exemple l'hydrure de potassium)

ou d'un alcoyllithium ou d'un aryllithium (par exemple le n-butyllithium ou le mésityllithium), dans un solvant organique tel qu'un éther (par exemple le dioxanne, le tétrahydrofuranne ou le diméthoxy-1,2 éthane) ou dans un mélange de solvants organiques comprenant un éther (tel que cité ci-dessus) et un solvant tel que l'hexaméthylphosphorotriamide, la N-méthylpyrrolidone ou la diméthyl-1,3 imidazolidinone-2, à une température comprise entre −78°C et la température de reflux du mélange réactionnel, éventuellement sous atmosphère inerte (azote ou argon).

Le cas échéant la réduction du S-oxyde s'effectue selon les méthodes décrites dans DE—A—2 637 176.

L'élimination des radicaux protecteurs d'amine, d'acides et/ou d'oxime s'effectue par toute méthode connue qui n'altère pas le reste de la molécule.

A titre d'exemple:

pour les groupements protecteurs d'amines:

lorsqu'il s'agit d'un radical benzyle, dibenzyle ou benzyloxycarbonyle: par hydrogénation catalytique,

lorsqu'il s'agit d'un radical p.nitrobenzyloxycarbonyle: par réduction, notamment par traitement par le zinc dans l'acide acétique ou par hydrogénolyse,

lorsque $R^{o}_1$ représente t.butoxycarbonyle, trityle ou p.méthoxybenzyloxycarbonyle: par traitement en milieu acide. De préférence on utilise l'acide trifluoroacétique en opérant à une température comprise entre 0 et 20°C, ou bien on utilise l'acide formique, phosphorique ou polyphosphorique purs ou en présence d'eau à température comprise entre 20 et 60°C, ou encore l'acide paratoluènesulfonique ou méthanesulfonique dans l'acétone ou l'acétonitrile à une température comprise entre 20°C et la température de reflux du mélange réactionnel,

pour les groupements protecteurs d'acide:

lorsqu'il s'agit d'un groupement t.butyle, p.méthoxybenzyle ou benzhydryle: par traitement en milieu acide, dans les conditions décrites ci-avant pour l'élimination du radical trityle protecteur d'amino. Dans le cas du radical benzhydryle, on peut opérer en présence d'anisole,

lorsqu'il s'agit d'un groupement méthoxyméthyle: par traitement en milieu acide dilué,

lorsqu'il s'agit d'un groupement nitrobenzyle: par réduction (notamment traitement par le zinc dans l'acide acétique ou hydrogénolyse).

Pour les radicaux protecteurs d'oxime:

lorsqu'il s'agit du radical trityle ou tétrahydropyrannyle: par acidolyse, par exemple par l'acide trifluoracétique, l'acide formique aqueux ou non ou l'acide paratoluènesulfonique,

lorsqu'il s'agit du radical méthoxy-2 propyle-2: selon la méthode décrite dans BE—A—875 379.

Les produits de formule générale (VII) pour lesquels n = 0 peuvent être obtenus par hydrolyse en milieu acide ou neutre de l'énamine de formule générale:

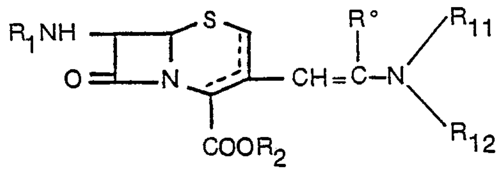

(IX)

ou un mélange de ses isomères [dans laquelle $R_1$ est défini comme $R°_{1a}$ en a) et c) à l'exception pour $R^c_5$ dans $R_5$ et pour $R_6$ de représenter un atome d'hydrogène, $R_2$ est un radical protecteur tel que défini précédemment pour $R°_{2a}$, R° est défini comme précédemment et les symboles $R_{11}$ et $R_{12}$, qui sont identiques ou différents, représentent des radicaux alcoyle (éventuellement substitués par un radical alcoyloxy ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, et qui se présente sous forme bicyclooctène-2 ou -3, les substituants R° et $—NR_{11}R_{12}$ de la chaîne en position -3 pouvant se trouver respectivement en position cis et trans (ou inversement) du bicyclooctène] puis élimination éventuelle des radicaux protecteurs d'acide $R^c_5$ et/ou $R_2$ (pour obtenir un produit pour lequel $R°_1$ contient un radical carboxy libre et/ou $R°_2$ est un atome d'hydrogène).

On opère de préférence dans un acide organique (par exemple acide formique, acide acétique) ou minéral (par exemple acide chlorhydrique, acide sulfurique) en présence ou non d'un solvant, en milieu aqueux ou organique, à une température comprise entre —20°C et la température de reflux du mélange réactionnel, puis on traite éventuellement par une base minérale (bicarbonate alcalin) ou organique (amine tertiare ou pyridine).

Lorsque l'on opère en milieu organique, l'hydrolyse est réalisée par addition d'eau au mélange réactionnel.

Lorsque l'on opère en présence d'un solvant, il n'est pas nécessaire que le solvant soit miscible à la phase aqueuse acide. Le contact est alors réalisé par agitation vive.

Parmi les solvants utilisables peuvent être cités les solvants chlorés, l'acétate d'éthyle, le tétrahydrofuranne, l'acétonitrile, le diméthylformamide les alcools.

Il n'est pas absolument nécessaire d'avoir purifié l'intermédiaire de formule générale (IX) pour mettre en oeuvre cette réaction.

Lorsque l'on veut préparer un produit de formule générale (VII) pour lequel $R°_2$ est un atome d'hydrogène, l'élimination des groupements protecteurs du radical carboxy s'effectue dans les conditions décrites précédemment.

Lorsque l'on veut préparer un produit de formule générale (VII) pour lequel $R°_2$ est un atome d'hydrogène et $R°_1$ est un radical de formule générale (II) dans laquelle $R_5$ est un groupement de formule générale (III) dont la fonction acide est protégée, il est nécessaire d'utiliser une ènamine de formule générale (IX) dans laquelle $R_2$ et $R^c_5$ sont différents et éliminables sélectivement. Il en est de même si l'on veut inversement préparer une cétone de formule générale (VII) dans laquelle $R°_1$ contient un groupement carboxy libre et $R°_2$ est autre qu'un atome d'hydrogène.

Les produits de formule générale (VII) pour lesquels $n = 1$ peuvent être obtenus par oxydation du produit de formule générale (VII) correspondant pour lequel $n = 0$. On opère par application des méthodes décrites dans DE—A—2 637 176.

Les produits de formule générale (VII) pour lesquels $R°_1$ est un radical de formule générale (II) contenant le cas échéant une fonction acide libre ou protégée, et $R°_2$, R° et n sont définis comme précédemment, peuvent être aussi obtenus par acylation d'une amino-7 céphalosporine (ou le cas échéant un mélange de ses isomères) de formule générale (VII) dans laquelle, R°, $R°_2$ et n étant définis comme ci-dessus, $R°_1$ est un atome d'hydrogène, au moyen d'un acide représenté par la formule générale:

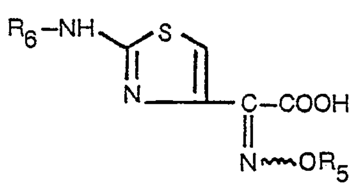

(X)

dans laquelle $R_5$ et $R_6$ sont définis comme dans la formule générale (II) à l'exception pour $R_5$, $R_6$ et pour $R^c_5$ dans $R_5$ de représenter un atome d'hydrogène, ou par action d'un dérivé réactif de cet acide, puis éventuellement élimination des radicaux protecteurs.

Il est entendu que, lorsque $R°_1$ contient un groupement hydroxyimino, celui-ci est libre ou protégé (par un groupement protecteur tel que cité précédemment pour $R_5$);

les fonctions acides sont protégées par un groupement protecteur tel que défini précédemment pour $R°_{2a}$.

Il est entendu que l'acide de formule générale (X) sous forme syn, anti ou leurs mélanges, conduit respectivement aux produits de formule générale (VII) de forme syn, anti ou leurs mélanges.

Généralement on effectue la condensation du produit de formule générale (X), dont la fonction acide est libre, sur l'amino-7 céphalosporine de formule générale (VII) dans un solvant organique tel que le diméthylformamide, l'acétonitrile, le tétrahydrofuranne, le chlorure de méthylène ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre –20 et 40°C.

Lorsque l'on utilise un dérivé réactif de l'acide de formule générale (X), il est possible de mettre en oeuvre l'anhydride, un anhydride mixte ou un ester réactif de formule générale:

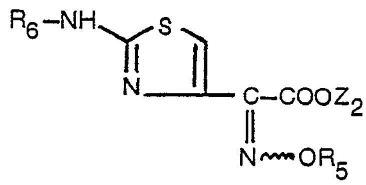

(XI)

[dans laquelle, $R_5$ et $R_6$ étant définis comme précédemment, $Z_2$ représente un radical succinimido, benzotriazolyle-1, nitro-4 phényle, dinitro-2,4 phényle, pentachlorophényle ou phtalimido]] ou bien un halogénure d'acide, par exemple le chlorure d'acide.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple l'acétone), ainsi que des mélanges de tels solvants [en présence d'un accepteur d'acide tel qu'un époxyde (par exemple l'oxyde de propylène) ou tel qu'une base organique azotée comme la pyridine, la diméthylaminopyridine, la N-méthylmorpholine, le NN diméthylacétamide ou une trialcoylamine (par exemple triéthylamine)] ou dans un milieu hydroorganique en présence d'un agent alcalin de condensation tel que le bicarbonate de sodium, et l'on opère à une température comprise entre –40 et +40°C.

Lorsque l'on met en oeuvre un ester réactif de formule générale (XI) on opère généralement en présence d'une trialcoylamine (par exemple triéthylamine) dans un solvant organique tel que le diméthylformamide, à une température comprise entre 0 et 40°C.

L'élimination des radicaux protecteurs s'effectue dans les conditions décrites précédemment.

L'amino-7 céphalosporine de formule générale (VII) dans laquelle $R°_1$ est un atome d'hydrogène, peut être obtenue par élimination du radical $R°_1$ d'un produit de formule générale (VII) dans laquelle $R°_1$ est défini comme $R°_{1a}$ en c) et $R°_2$ est défini comme précédemment, ou bien par élimination simultanée des radicaux $R°_1$ et $R°_2$ d'un produit de formule générale (VII) dans laquelle $R°_1$ et $R°_2$ sont définis comme ci-dessus.

L'élimination des radicaux protecteurs s'effectue par toute méthode connue pour la libération d'une fonction amine et/ou acide qui n'altère pas le reste de la molécule.

A titre d'exemple:

L'élimination des groupements protecteurs d'amines s'effectue soit selon l'une des méthodes citées précédemment, soit

lorsque $R°_1$ représente un radical diphénylphosphinoyle: selon la méthode décrite par P. HAAKE et coll., J. Am. Chem. Soc., *95*, 8073 (1973)

lorsque $R°_1$ représente un radical $(Z'O)_2P(O)$—: selon la méthode décrite dans BE—A—833 619 ou, lorsque Z' est trichloro-2,2,2 éthyle ou p.nitrobenzyle, par réduction (notamment par le zinc dans l'acide acétique).

lorsque $R°_1$ représente phénylacétyle ou phénoxyacétyle: selon la méthode décrite dans le brevet belge 758 800,

lorsque $R°_1$ représente diphénylméthoxycarbonyle, (biphénylyl-4)-2 isopropyloxycarbonyle ou vinyloxycarbonyle et lorsque $R°_1NH$— est remplacé par diméthylaminométhylèneamino, diméthoxy-3,4 benzylidèneamino ou nitro-4 benzylidèneamino: par hydrolyse en milieu acide.

Les acides de formule générale (X) dans laquelle $R_5$ est hydrogène ou alcoyle peuvent être préparés selon la methode déc décrite dans BE—A—850 662.

Les produits de formule générale (X) dans laquelle $R_5$ est un radical vinyle peuvent être préparés selon la methode decrite dans BE—A—869 079.

Les produits de formule générale (X) dans laquelle $R_5$ est un radical cyanométhyle peuvent être préparés selon la méthode décrite dans la demande de brevet allemand 2 812 625.

Les acides de formule générale (X) dans laquelle $R_5$ est un radical protecteur, peuvent être préparés par protection de l'oxime d'un tel acide dans lequel $R_5$ est hydrogène, par toute méthode connue qui n'altère pas le reste de la molécule. La protection s'effectue notamment par les groupements trityle ou tétrahydropyrannyle qui sont éliminables par acidolyse, par exemple par l'acide trifluoracétique, l'acide formique (aqueux ou non) ou l'acide p.toluènesulfonique. La protection peut

également s'effectuer par le groupement méthoxy-2 propyle-2 qui peut être éliminé par application de la méthode décrite dans BE—A—875 379.

Les acides de formule générale (X) dans laquelle $R_5$ est un radical de formule générale (III) peuvent être préparés selon les méthodes décrites dans BE—A—864 810, BE—A—865 298, BE—A—876 541 et BE—A—876 542.

Les produits de formule générale (IX) pour lesquels $R_1$, $R_2$, $R_{11}$ et $R_{12}$ ont les définitions données précédemment et R° est un radical du premier groupe défini précédemment peuvent être obtenus par action d'un réactif, éventuellement préparé in situ, de formule générale:

$$R^o—C(NR_{11}R_{12})_3 \qquad (XII)$$

dans laquelle R°, $R_{11}$ et $R_{12}$ sont définis comme ci-dessus, sur un dérivé de céphalosporine de formule générale:

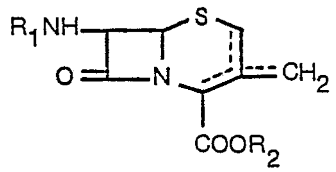

(XIII)

dans laquelle, $R_1$ et $R_2$ étant définis comme précédemment, le dérivé se présente sous forme méthyl-3 bicyclooctène-2 ou -3 ou méthylène-3 bicyclooctane.

On opère généralement dans un solvant organique tel que le diméthylformamide, l'hexaméthylphosphorotriamide, les glymes, le dioxanne, le diméthylacétamide, l'acétate d'éthyle ou dans un mélange de tels solvants à une température comprise entre 20°C et la température de reflux du mélange réactionnel.

Les produits de formule générale (IX) dans laquelle $R_1$, $R_2$, $R_{11}$ et $R_{12}$ sont définis comme précédemment et R° est un radical du second groupe défini précédemment peuvent être obtenus par action d'un réactif, éventuellement préparé in situ, de formule générale:

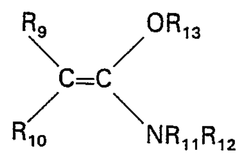

(XIVa)

ou

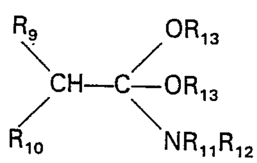

(XIVb)

(dans lesquelles $R_9$ et $R_{10}$ sont tels que $R_9R_{10}CH—$ représente le radical R° défini ci-dessus, $R_{11}$ et $R_{12}$ sont définis comme précédemment et $R_{13}$ est un radical alcoyle contenant 1 à 5 atomes de carbone), sur un dérive de céphalosporine de formule générale (XIII).

La réaction s'effectue généralement dans les conditions décrites précédemment pour la préparation des produits de formule générale (IX) à partir des produits (XII) et (XIII).

Les réactifs de formule générale (XII) peuvent être obtenus selon ou par application de la méthode décrite par C. F. HOBBS et coll., J. Org. Chem. *36,* 2885 (1971).

Les réactifs de formule générale (XIVa) peuvent être obtenus selon ou par application de la méthode décrite par H. BREDERECK et coll., Chem. Ber., *97,* 3076 (1964) et Chem. Ber., *97* 3081 (1964).

Les réactifs de formule générale (XIVb) peuvent être obtenus par application de la méthode décrite par H. MERWEIN et coll., Ann., *641,* 1 (1961).

Les dérivés de la céphalosporine de formule générale (XIII) dans laquelle $R_1$ représente un radical de formule générale (II) peuvent être préparés à partir des produits de formule générale:

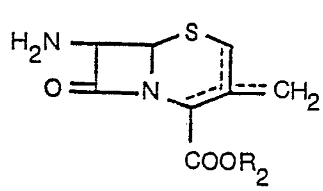

(XV)

[dans laquelle, $R_2$ étant défini comme précédemment, la position de la double liaison est définie comme pour le produit de formule générale (XIII)] par action d'un acide de formule générale (X) [dans laquelle $R_5$ et $R_6$ sont définis comme précédemment pour la formule générale (X) à l'exception pour $R_5$ de représenter l'atome d'hydrogène] ou d'un dérivé réactif de cet acide, suivie le cas échéant de l'élimination du radical protecteur de l'oxime.

On opère par analogie avec la méthode décrite précédemment pour la préparation d'un produit de formule générale (VII) à partir d'un produit de formule générale (X) et d'un produit de formule générale (VII) dans laquelle $R°_1$ est un atome d'hydrogène.

L'introduction des groupements protecteurs $R_1$ et/ou $R_2$ des produits de formule générale (XIII), pour lesquels $R_1$ et $R_2$ sont définis comme précédemment [à l'exception pour $R_1$ de représenter un radical de formule générale (II)], et des produits de formule générale (XV), pour lesquels $R_2$ est défini comme précédemment, peut être effectuée sur une céphalosporine respectivement de formule générale (XV) ou de formule générale:

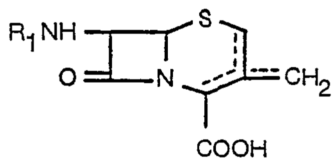

(XVI)

ou

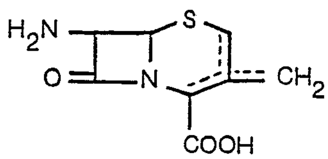

(XVII)

par application des méthodes décrites dans les références suivantes:

lorsque $R_1$ est un radical trityle: par analogie avec la méthode décrite par J. C. SHEEHAN et coll., J. Amer. Chem. Soc., *84,* 2983 (1962),

lorsque $R_1$ est phénylacétyle ou phénoxyacétyle: selon E. H. FLYNN, Cephalosporins and Penicillins, Ac. Press (1972),

lorsque $R_1$ est un radical t.butoxycarbonyle: selon L. MORODER et coll., Hoppe Seyler's Z. Physiol. Chem. *357,* 1651 (1976),

lorsque $R_1$ est benzyloxycarbonyle, p.méthoxybenzyloxycarbonyle, p.nitrobenzyloxycarbonyle ou vinyloxycarbonyle: par action d'un chloroformiate en milieu hydroorganique en présence d'un bicarbonate alcalin, ou selon BE—A—788 885.

lorsque $R_1$ est diphénylméthoxycarbonyle: par action de l'azidoformiate correspondant en milieu hydroorganique, en présence d'un bicarbonate alcalin,

lorsque $R_1$ est (biphénylyl-4)-2 isopropyloxycarbonyle: par analogie avec la méthode décrite dans Helv. Chim. Acta. *51,* 924 (1968),

lorsque $R_1$ est un radical de formule générale (IVc): par application de la méthode décrite par A. MORIMOTO et coll., J. C. S. Perkin I, 1109 (1980) à partir des halogénures correspondants [qui peuvent être eux-mêmes obtenus selon Houben Weyl, Methoden der Organischen Chemie, vol. 12 part 2, page 274, Georg Thieme Verlag Stuttgart (1964)],

lorsque $R_1NH$ est remplacé par diméthylamino méthylèneamino: par analogie avec la méthode décrite par J. F. FITT, J. Org. Chem. *42 (15),* 2639 (1977),

lorsque $R_1NH$ est remplacé par nitro-4 benzylidèneamino ou diméthoxy-3,4 benzylidèneamino: selon la méthode décrite par R. A. FIRESTONE, Tetrahedron Lett., 375 (1972),

lorsque $R_2$ est méthoxyméthyle: selon S. SEKI et coll., Tetrahedron Lett., *33,* 2915 (1977),

lorsque $R_2$ est t.butyle: selon R. J. STEDMAN, J. Med. Chem., *9,* 444 (1966),

lorsque $R_2$ est benzhydryle: selon NL—A—73 03263,

lorsque $R_2$ est benzyle, nitrobenzyle ou p.méthoxybenzyle: selon R. R. CHAUVETTE et coll., J. Org. Chem., *38 (17),* 2994 (1973).

II) Selon l'invention les céphalosporines de formule générale (I) dans laquelle $R°_{2a}$, $R_3$ et n sont définis comme précédemment et $R°_{1a}$ représente un atome d'hydrogène peuvent être obtenus par élimination du radical protecteur $R°_{1a}$ ou éventuellement élimination simultanée des radicaux protecteurs $R°_{1a}$ et $R°_{1a}$ et $R°_{2a}$ d'un produit de formule générale (I) [dans laquelle $R°_{1a}$ est défini comme précédemment en c)].

On opère dans les conditions décrites précédemment pour la préparation d'un produit de formule générale (VII) dans laquelle $R°_1$ est un atome d'hydrogène.

III) Selon l'invention les produits de formule générale (I) dans laquelle $R°_{2a}$, $R_3$ et n sont définis comme précédemment, et $R°_{1a}$ est un radical de formule générale (II) tel que défini en a), peuvent être préparés par acylation d'une amino-7 céphalosporine de formule générale (I) dans laquelle $R°_{1a}$ est un atome d'hydrogène (ou le cas échéant d'un mélange de ses isomères), au moyen d'un acide de formule

générale (X) ou d'un dérivé réactif de cet acide, puis éventuellement réduction du sulfoxyde obtenu et éventuellement élimination des radicaux protecteurs.

On opère dans les conditions décrites précédemment pour la préparation d'un céphalosporine de formule générale (VII), les conditions de blocage étant les mêmes.

Le cas échéant la réduction du sulfoxyde et l'élimination des radicaux protecteurs d'acide et/ou d'amine s'effectuent dans les conditions décrites précédemment.

IV) Selon l'invention, les produits de formule générale (I) dans laquelle n = 1 peuvent être obtenus par oxydation des produits correspondants dans lesquels n = 0 selon la méthode décrite dans DE—A—2 637 176.

Les nouveaux produits de formule générale (I) sont des intermédiaires pour la préparation de produits de formule générale:

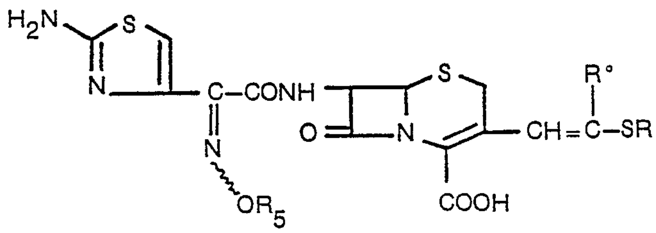

(XVIII)

doués d'activité antibactérienne, qui font l'objet de la demande EP—A—53542.

Dans la formule générale (XVIII), R° est défini comme précédemment, $R_5$ est un atome d'hydrogène, un radical alcoyle, vinyle ou cyanométhyle ou représente un groupement de formule générale (III) (dans laquelle, $R^a_5$ et $R^b_5$ étant définis comme précédemment, $R^c_5$ est un atome d'hydrogène) et R est choisi parmi les significations:

1) pyridyle-2, -3 ou -4 éventuellement N-oxydés

2) pyrimidinyle-2

3) méthyl-6 oxyde-1 pyridazinyle-3

4) dioxo-5,6 tétrahydro-1,4,5,6 triazine-1,2,4 yle-3 substitué en position -4 par

a) un radical alcoyle contenant 1 ou 2 atomes de carbone éventuellement substitué par un radical alcoyloxy, alcoylthio, formyle,

b) un radical allyle, dihydroxy-2,3 propyle, dihydroxy-1,3 propyle-2 ou formyl-2 hydroxy-2 éthyle,

c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,

5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle,

6) alcoyl-2 dihydro-2,5 oxo-5 triazine-1,2,4 yle-3 éventuellement substitué en position -6 par un radical alcoyle ou alcoyloxy dont les portions et radicaux alcoyles contiennent 1 ou 2 atomes de carbone,

7) amino-1 dihydro-1,2 oxo-2 pyrimidinyle-4

8) thiadiazol-1,3,4 yle-5 substitué par alcoyle, dialcoylaminoalcoyle ou acylaminoalcoyle,

9) tétrazolyle-5 substitué en position -1 par

a) un radical alcoyle contenant 1 ou 2 atomes de carbone éventuellement substitué par un radical formyle,

b) un radical alcoyle contenant 2 ou 3 atomes de carbone substitué par hydroxy, acylamino, ou dialcoylamino, ou

10a) alcoyl-1 triazol-1,2,4 yle-5 éventuellement substitué en position-3 par un radical alcoyloxycarbonyle dont les radicaux alcoyle et alcoyloxy contiennent 1 ou 2 atomes de carbone

b) alcoyl-1 triazol-1,3,4 yle-5

Il est entendu que les substituants R° et —SR peuvent être situés respectivement en position E et Z ou inversement par rapport au noyau céphalosporine et que ces isomères et leurs mélanges entrent dans la définition de la formule générale (XVIII).

I°-) Les thiovinyl-3 céphalosporines dont la préparation est decrite plus en détails dans EP—A—53542 de formule générale (XVIII) peuvent être préparées par action d'un thiol (ou d'un de ses sels alcalins alcalino-terreux) de formule générale:

$$R—SH \qquad (XIX)$$

dans laquelle R, qui est défini précédemment, est éventuellement protégé, sur un dérivé de la céphalosporine (ou un mélange des isomères) de formule générale (I) dans laquelle $R°_{1a}$ est défini comme en a), suivie de la réduction du sulfoxyde obtenu lorsque n = 1 et le cas échéant de l'élimination des radicaux protecteurs.

Lorsque l'on veut obtenir un produit de formule générale (XVIII) dans laquelle R contient un radical

formyle, on met en oeuvre un thiol de formule générale (XIX) dans laquelle ce radical est protégé à l'état d'acétal de formule générale:

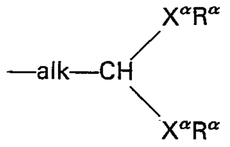

(XX)

ou 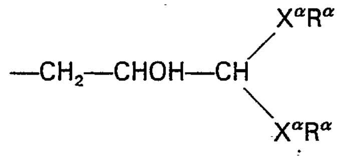

(XXI)

dans lesquelles alk est un radical alcoylène contenant 1 ou 2 atomes de carbone, $X^\alpha$ et $Y^\alpha$ sont identiques et représentent des atomes d'oxygène ou de soufre et $R^\alpha$ représente un radical alcoyle, ou bien $X^\alpha$ et $Y^\alpha$ sont identiques ou différents et représentent des atomes d'oxygène ou de soufre et les radicaux $R^\alpha$ forment ensemble un radical alcoylène contenant 2 ou 3 atomes de carbone.

Les thiols de formule générale (XIX), qui peuvent être mis en oeuvre sous leur forme tautomère, peuvent être préparés par application des méthodes décrites dans la littérature, et dont le détail est donné EP—A—53542.

II°-) Les thiovinylcéphalosporines de formule générale (XVIII) peuvent également être obtenues de la manière suivante:

On fait agir un thiol de formule générale (XIX) (ou un de ses sels alcalins ou alcalino-terreux) sur un produit ou un mélange d'isomères du produit de formule générale (I) dans laquelle $R^\circ_{1a}$ est défini comme précédemment en c) puis on réduit éventuellement le sulfoxyde obtenu (lorsque n = 1) et on élimine éventuellement les radicaux protecteurs de R pour préparer un produit de formule générale:

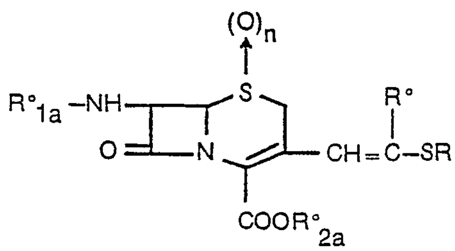

(XXII)

dans laquelle, n étant comme précédemment, R°, $R^\circ_{1a}$ et $R^\circ_{2a}$ sont définis comme ci-dessus et R est défini comme précédemment.

On prépare un produit de formule générale:

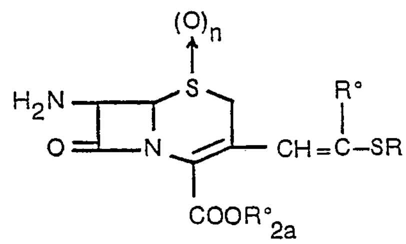

(XXIII)

dans laquelle R, R°, $R^\circ_{2a}$ et n sont définis comme ci-dessus, par élimination du radical $R^\circ_{1a}$ d'un produit de formule générale (XXII) tel que défini précédemment ou éventuellement élimination simultanée du radical $R^\circ_{1a}$ et des autres radicaux protecteurs de ce produit.

Il n'est pas nécessaire d'isoler le produit de formule générale (XXIII) pour l'utiliser dans la suite de la synthèse.

On prépare alors la thiovinyl-3 céphalosporine de formule générale (XVIII) dans laquelle R, R°, $R^\circ_{1a}$ et $R^\circ_{2a}$ sont définis comme précédemment, par acylation d'une amino-7 céphalosporine de formule générale (XXIII) au moyen d'un acide représenté par la formule générale (X) [dans laquelle $R_5$ et $R_6$ sont définis comme précédemment], ou d'un dérivé réactif de cet acide, dans les conditions décrites précédemment pour la préparation des produits de formule générale (XIII), puis on réduit l'oxyde obtenu (lorsque n = 1) et élimine les radicaux protecteurs.

Les produits de formule générale (XXII) dans laquelle n = 1 peuvent être obtenus par oxydation des produits correspondants dans lesquels n = 0 selon la méthode décrite dans DE—A—2 637 176.

Les isomères des produits de formules générales (I), (VII), (IX), (XVIII), (XXII) et (XXIII) peuvent être séparés par chromatographie ou par crystallisation.

Plus particulièrement, les isomères bicyclooctène-2 des produits de formules générales (I), (VII), (XVIII) ou (XXIII) peuvent être obtenus à partir des mélanges bicyclooctène-2/bicyclooctène-3, par oxydation puis réduction.

Les nouveaux produits de formule générale (I) dans laquelle existe un radical amino peuvent être transformés en sels d'addition avec les acides. Selon les procédés indiqués, les produits sont éventuellement obtenus sous forme de trifluoroacétate, paratoluènesulfonate, méthanesulfonate, phosphate ou solvate avec l'acide formique. Les produits obtenus sous forme de ces sels peuvent être libérés et transformés en sels d'autres acides selon les méthodes habituelles.

Les produits de formule générale (I) qui contiennent un radical carboxy peuvent aussi être transformés en sels métalliques ou en sels d'addition avec les bases organiques azotées selon les méthodes connues en soi. Ces sels peuvent être obtenus par action d'une base métallique (par exemple alcaline ou alcalino-terreuse) ou d'une amine sur un produit de formule générale (I) dans un solvant approprié tel qu'un alcool, un éther ou l'eau ou par réaction d'échange avec un sel d'un acide organique. Le sel formé précipite après concentration éventuelle de sa solution, il est séparé par filtration ou décantation.

Comme exemples de sels peuvent être cités les sels avec les métaux alcalins (tels que les sels de potassium, de sodium ou de lithium) ou avec les métaux alcalino-terreux, les sels de bases azotées (sels de diméthylamine, de diéthylamine, de diisopropylamine, de dicyclohexylamine, de N-éthylpipéridine et de N-méthylmorpholine) et les sels d'addition avec les acides minéraux (tels que chlorhydrates ou bromhydrates) ou organiques (formiates, trifluoracétates, p.toluènesulfonates, naphthalènesulfonates ou oxalates).

Les dérivés de la céphalosporine de formule générale (XVIII) et leurs sels pharmaceutiquement acceptables présentent des propriétés antibactériennes particulièrement intéressantes. Ils manifestent une activité remarquable in vitro et in vivo sur les germes Gram-positifs et Gram-négatifs.

D'un intérêt particulier sont les produits de formule générale (I) pour lesquels
n = 0 ou 1,
le symbole $R^{o}_{1a}$ est un atome d'hydrogène ou un radical de formule générale (IVb),
le symbole $R^{o}_{2a}$ est un radical protecteur
le symbole $R^{o}$ est un radical alcoyle, et
le symbole $R_3$ est un radical de formule générale (V) dans laquelle $R_4$ est un radical alcoyle, trihalogénométhyle, ou phényle éventuellement substitué par un radical alcoyle.

Parmi ces produits, plus spécialement intéressants sont les produits de formule générale (I) pour lesquels n = 0 ou 1, $R^{o}_{1a}$ est un atome d'hydrogène ou un radical de formule générale (IVb) dans laquelle $R_8$ est un radical alcoyle ramifié, $R^{o}_{2a}$ est un radical benzhydryle, $R^{o}$ est un radical méthyle et $R_3$ est un radical de formule général (V) dans laquelle $R_4$ est alcoyle, trifluorométhyle ou phényle substitué par un radical méthyle.

Les exemples suivants, donnés à titre non limitatif, illustrent la présente invention.

Exemple 1

A une solution de 6,6 g de tétraméthyl-2,2,6,6 pipéridine dans 50 cm3 de tétrahydrofuranne à 20°C on ajoute en 1 minute 26,5 cm3 de solution 1,6 M de n.butyllithium dans l'hexane. On agite pendant 30 minutes à 20°C puis refroidit le mélange à −70°C. On ajoute goutte en 20 minutes une solution de 22,2 g d'acétonyl-3 benzhydryloxycarbonyl-2 t.butoxycarbonylamino-7 oxo-8 thia-5 aza-1 bicyclo[4.2.0] octène-2 dans 50 cm3 de tétrahydrofuranne. On agite pendant 30 minutes à −70°C et ajoute 35 cm3 d'hexaméthylphosphorotriamide en 3 minutes puis 10,7 cm3 d'anhydride trifluorométhanesulfonique en 5 minutes. On agite pendant 45 minutes à −70°C puis laisse revenir à 0°C en 1 heure. Le mélange est alors dilué avec 500 cm3 d'acétate d'éthyle et lavé successivement avec 650 cm3 d'acide chlorhydrique 0,2N et 300 cm3 d'eau distillée. La solution est séchée sur sulfate de sodium et concentrée à sec sous pression réduite (20 mm de mercure; 2,7 kPa) à 20°C. Le résidu est chromatographié sur une colonne de 300 g de gel de silice Merck (0,06—0,2) (diamètre de la colonne: 3,5 cm, hauteur: 70 cm). On élue avec 2 litres de mélange acétate d'éthyle/cyclohexane 20—80 (volumes) et recueille des fractions de 100 cm3. On concentre à sec sous pression réduite (20 mm de mercure; 2,7 kPa) à 20°C les fractions 3 à 9. On obtient ainsi 6,3 g du mélange 66—34 des benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène et octène-3, forme Z sous forme d'une huile jaune.

Spectre infra-rouge ($CHCl_3$), bandes caractéristiques ($cm^{-1}$) 3440, 1790, 1720, 1505, 1455, 1420, 1370, 1155, 1140, 910, 700.

Spectre de RMN du proton (350 MHz, $CDCl_3$, $\delta$ en ppm, J en Hz) 1,47 (s, 9H, $—(CH_3)_3$);

1,94 (s, 3H, $CH_3—\overset{|}{C}=$ de l'octène-2);

1,99 (s, 3H, $CH_3—\overset{|}{C}=$ de l'octène-3);

3,43 et 3,72 (2d, J = 18, 2H, —$SCH_2$—); 5,00 (d, J = 4, $H_6$ octène-2); 5,04 (s, $H_2$ de l'octène-3); 5,26 (d, J = 4, $H_6$ octène-3); 5,27 (d, J = 9, —CONH— octène-2); 5,35 à 5,48 (m, 2H, $H_7$ et —CONH— de l'octène-3); 5,56 (s, $H_4$ de l'octène-3); 5,65 (dd, J = 4 et 9, $H_7$ de l'octène-2);

6,17 (s, 1H, —CH=C— de l'octène-2);
|

6,65 (s, 1H, —CH=C— de l'octène-3);
|

6,87 (s, 1H, —COOCH< octène-3);

6,95 (s, 1H, —COOCH< de l'octène-2).

L'acétonyl-3 benzhydryloxycarbonyl-2 t.butoxycarbonylamino-7 oxo-8 thia-5 aza-1 bicyclo[4.2.0] octène-2 peut être préparé de la manière suivante:

A une solution de 345 g de benzhydryloxycarbonyl-2 t.butoxycarbonylamino-7 méthyl-3 oxo-8 thia-5 aza-1 bicyclo[4.2.0] octène-2 dans 1600 cm3 de diméthylformamide anhydre à 80°C on ajoute goutte à goutte en 55 minutes 218 g de diméthylamino-1 méthoxy-1 éthylène. On obtient ainsi une solution de benzhydryloxy carbonyl-2 t.-butoxycarbonylamino-7 (diméthylamino-2 propène-1 yl-1)-3 oxo-8 thia-5 aza-1 bicyclo[4.2.0] octène-2 que l'on verse dans un mélange agité de 2 litres d'eau distillée, 2 kg de glace et 2,5 litres d'acétate d'éthyle. La phase organique est décantée, lavée avec 3 fois 1 litre d'eau distillée, séchée sur sulfate de sodium et concentrée à sec sous pression réduite (20 mm de mercure; 2,7 kPa) à 30°C. le résidu est partagé en 2 parties égales que l'on chromatographie séparément chacune sur une colonne de 1500 g de gel de silice Merck (0,06—0,2) (diamètre de la colonne: 8,5 cm; hauteur: 70 cm). On élue chaque colonne avec 20 litres de mélange acétate d'ethylecyclohexane 23—77 (volumes) en recueillant des fractions de 1 litre. On concentre à sec à 30°C sous pression réduite (20 mm de mercure; 2,7 kPa) les fractions 7 à 15 de chaque colonne. On obtient ainsi 149 g d'acétonyl-3 benzhydryloxycarbonyl-2 t.butoxycarbonylamino-7 oxo-8 thia-5 aza-1 bicyclo[4.2.0] octène-2 sous la forme d'une merinque jaune.

Spectre infra-rouge ($CHCl_3$), bandes caractéristiques ($cm^{-1}$) 3440, 1780, 1720, 1505, 1455, 1395, 1370, 1160, 695.

Spectre de RMN du proton (350 MHz, $CDCl_3$, $\delta$ en ppm, J en Hz) 1,48 (s, 9H, —$C(CH_3)_3$); 2,10 (s, 3H, $CH_3CO$—); 3,29 et 3,62 (2d, J = 19, 2H, —$SCH_2$—); 3,57 et 3,67 (2d, J = 16, 2H, —$CH_2CO$—); 5,00 (d, J = 4, 1H, $H_6$); 5,23 (d, J = 9, 1H, —CONH—); 5,62 (dd, J = 4 et 9, 1H, $H_7$); 6,97

(s, 1H, —COOCH< ).

Exemple 2

A une solution de 1,28 g de tétramethyl-2,2,6,6 pipéridine dans 10 cm3 de tétrahydrofuranne à 20°C on ajoute en 2 minutes 5,37 cm3 d'une solution 1,6M de n.butyllithium dans l'hexane. Le mélange est agité pendant 1 heure à 20°C puis refroidi à —70°C. On ajoute alors goutte à goutte en 20 minutes une solution de 4,5 g d'acétonyl-3 benzhydryloxycarbonyl-2 t.butoxycarbonylamino-7 oxo-8 thia-5 aza-1 bicyclo[4.2.0]octène-2 dans 10 cm3 de tétrahydrofuranne puis agite la solution pendant 30 minutes à —70°C. On ajoute alors 7 cm3 d'hexaméthylphosphorotriamide en 3 minutes puis 4,34 cm3 d'anhydride trifluorométhanesulfonique en 3 minutes. On agite encore pendant 40 minutes à —70°C puis laisse revenir à 0°C en 40 minutes. Le mélange est alors versé dans un mélange agité de 125 cm3 d'acide chlorhydrique 0,25 N et de 50 cm3 d'acétate d'éthyle. La phase organique est décantée, lavée avec 50 cm3 de solution aqueuse saturée de bicarbonate de sodium, séchée sur sulfate de sodium et concentrée à sec sous pression réduite (20 mm de mercure; 2,7 kPa) à 20°C. Le résidu est chromatographié sur une colonne de 90 g de gel de silice Merck (0,06—0,20) (diamètre de la colonne: 2,4 cm; hauteur: 45 cm). On élue avec 1 litre de mélange acétate d'éthyle/cyclohexane 20—80 (en volumes) et recueille des fractions de 100 cm3. On concentre à sec les fractions 2 à 7 sous pression réduite (20 mm de mercure; 2,7 kPa) à 20°C. On obtient ainsi 2 g de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2, forme Z sous forme d'une meringue jaune.

Spectra infra-rouge ($CHCl_3$), bandes caractéristiques ($cm^{-1}$) 3440, 1790, 1720, 1505, 1455, 1420, 1370, 1155, 1140, 910, 700.

Spectre de RMN du proton (350 MHz, $CDCl_3$, $\delta$ en ppm, J en Hz) 1,47 (s, 9H, —$C(CH_3)_3$); 1,94

(s, 3H, $CH_3$—C=C—);

3,43 et 3,72 (2d, J = 18, 2H, —$SCH_2$—); 5,00 (d, J = 4, $H_6$); 5,27 (d, J = 9, —CONH—); 5,65 (dd, J = 4 et 9, $H_7$); 6,17

(s, 1H, —CH=C—);

6,95 (s, 1H, —COOCH< ).

Exemple 3

A une solution de 0,78 g de tétraméthyl-2,2,6,6 pipéridine dans 5 cm3 de tétrahydrofuranne à 20°C on ajoute en 10 minutes 3,44 cm3 de solution 1,6 M de butyllithium dans l'hexane. On agite la solution pendant 10 minutes à 20°C puis on refroidit à —70°C. On ajoute alors en 20 minutes une solution de 2,54 g d'acétonyl-3 benzhydryloxycarbonyl-2 t.butoxycarbonylamino-7 oxo-8 thia-5 aza-1 bicyclo[4.2.0] octène-2 dans 6 cm3 de tétrahydrofuranne puis on agite pendant 15 minutes à —70°C. On ajoute successivement 7 cm3 d'hexaméthylphosphorotriamide en 3 minutes et une solution de 5,23 g de chlorure de p.toluènesulfonyle dans 7 cm3 de tétrahydrofuranne en 3 minutes. On agite pendant 45 minutes à —70°C puis laisse remonter la température jusqu'à 0°C, dilue la solution avec 100 cm3 d'acétate d'éthyle et lave successivement avec 50 cm3 de solution 0.1, N d'acide citrique, 50 cm3 de solution aqueuse saturée de bicarbonate de sodium et 100 cm3 d'eau distillée. La phase organique est séchée sur sulfate de sodium et concentrée à sec sous pression réduite (20 mm de mercure; 2,7 kPa) à 20°C. Le résidu est chromatographié sur une colonne de 110 g de gel de silice Merck (0,06—0,20) (diamètre de la colonne: 3 cm; hauteur: 26 cm). On élue avec 1200 cm3 d'un mélange acétate d'éthyle/cyclohexane 15—80 (en volumes) en recueillant des fractions de 60 cm3. On concentre à sec les fractions 3 à 15 sous pression réduite (20 mm de mercure; 2,7 kPa) à 20°C. On obtient ainsi 0,94 g de benzhydryloxycarbonyl-2 t.butoxycarbonylamino-7 (tosyloxy-2 propène-1 yl-1)-3 oxo-8 thia-5 aza-1 bicyclo[4.2.0] octène-2, forme Z sous forme d'une meringue jaune pâle.

Spectre infra-rouge ($CHBr_3$), bandes caractéristiques ($cm^{-1}$) 3420, 1785, 1720, 1595, 1500, 1455, 1370, 815, 530.

Spectre de RMN du proton (350 MHz, $CDCl_3$, $\delta$ en ppm, J en Hz) 1,5 (s, 9H, —$C(CH_3)_3$); 2,0

(s, 3H, $CH_3$—C=);

2,35 (s, 3H, —$CH_3$ tosyle); 3,51 (s large, 2H, —$SCH_2$—); 4,78 (d, J = 4, 1H, $H_6$); 5,92 (dd, J = 4 et 9, 1H, $H_7$); 6,08 (s, 1H, —CH=); 6,93

(s, 1H, —COOCH< ).

Exemple 4

A une solution de 2,33 g de tétraméthyl-2,2,6,6 pipéridine dans 15 cm3 de tétrahydrofuranne à 20°C, on ajoute en 10 minutes 10,3 cm3 de solution 1,6 M de butyllithium dans l'hexane. On agite pendant 20 minutes à 20°C puis on refroidit à —70°C. On ajoute alors en 15 minutes une solution de 7,61 g d'acétonyl-3 benzhydryloxycarbonyl-2 t.butoxycarbonylamino-7 oxo-8 thia-5 aza-1 bicyclo[4.2.0] octène-2 dans 20 cm3 de tétrahydrofuranne. On agite encore pendant 20 minutes à —70°C puis on ajoute 15 cm3 d'hexaméthylphosphorotriamide en 3 minutes et ensuite une solution de 4,5 cm3 de chlorure de méthanesulfonyle dans 10 cm3 de tétrahydrofuranne en 3 minutes. On agite pendant 45 minutes à —70°C puis laisse remonter la température à 0°C et dilue le mélange avec 300 cm3 d'acétate d'éthyle. La solution est lavée successivement avec 100 cm3 de solution saturée de bicarbonate de sodium et 100 cm3 d'eau distillée, séchée sur sulfate de sodium et concentrée à sec sous pression réduite (20 mm de mercure; 2,7 kPa) à 20°C. Le résidu est chromatographié sur une colonne de 150 g de gel de silice Merck (0,06—0,20) (diamétre de la colonne: 3,7 cm; hauteur: 30 cm). On élue avec 2,5 litres de mélange acétate d'éthyle/cyclohexane 22—78 (en volumes) en recueillant des fractions de 100 cm3. On concentre à sec sous pression réduite (20 mm de mercure; 2,7 kPa) à 20°C les fractions 12 à 20. On obtient ainsi 3,2 g de benzhydryloxycarbonyl-2 t.butoxycarbonylamino-7 (mésyloxy-2 propène-1 yl-1)-3 oxo-8 thia-5 aza-1 bicyclo[4.2.0] octène-2, forme Z sous forme d'une meringue jaune pâle.

Spectre infra-rouge ($CHCl_3$), bandes caractéristiques ($cm^{-1}$) 3430, 1790, 1720, 1610, 1500, 1455, 1375, 1155, 970.

Spectre de RMN du proton (350 MHz, $CDCl_3$, $\delta$ en ppm, J en Hz) 1,50 (s, 9H, —$C(CH_3)_3$); 1,80

(s, 3H, $CH_3$—C=);

3,15 (s, 3H, —$OSO_2CH_3$); 3,43 et 3,77 (2d, J = 18, 2H, —$SCH_2$—); 5,06 (s, J = 4, 1H, $H_6$); 5,38 (d, J = 9, 1H, —CONH—); 5,74 (dd, J = 4 et 9, 1H, $H_7$); 6,97

(s, 1H, —COOCH< ).

Exemple 5

A une solution de 0,42 g de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z dans 10 cm3 de dichlorométhane à 0°C on ajoute en 20 minutes une solution de 0,13 g d'acide m.chloroperbenzoïque à 85% dans 5 cm3 de dichlorométhane. On agite à 20°C pendant 10 heures puis lave la solution avec 20 cm3 de solution saturée de bicarbonate de sodium et sèche sur sulfate de sodium. On concentre à sec sous pression réduite (20 mm de mercure; 2,7 kPa) à 20°C et chromatographie le résidu sur une colonne de 6 g de gel de silice Merck (0,06—0,20) (diamètre de la colonne: 1 cm; hauteur: 12 cm). On élue avec 100 cm3 de mélange acétate d'éthyle/cyclohexane 30—70 (en volumes) en recueillant des fractions de 3 cm3. On concentre à sec les fractions 8 à 18 sous pression réduite (20 mm de mercure; 2,7 kPa) à 20°C. On obtient ainsi 0,14 g de benzhydryloxycarbonyl-2 t.butoxycarbonylamino-7 oxo-8 oxyde-5 (trifluorométhanesulfonyloxy-2 propène-1 yl-1)-3 thia-5 aza-1 bicyclo[4.2.0] octène-2, forme Z sous forme de meringue blanche.

Spectre infra-rouge ($CHBr_3$), bandes caractéristiques ($cm^{-1}$) 3420, 1810, 1720, 1505, 1455, 1420, 1220, 1040.

Spectre de RMN du proton (350 MHz, $CDCl_3$, $\delta$ en ppm, J en Hz) 1,50 (s, 9H, —$C(CH_3)_3$); 1,98

(s, 3H, $CH_3$—C=);

3,52 et 3,82 (2d, J = 18, 2H, —$SCH_2$—); 4,59 (d, J = 4, 1H, $H_6$); 5,73 (d, J = 8, 1H, —CONH—); 5,86 (dd, J = 4 et 8, 1H, $H_7$); 6,44 (s, 1H, —CH=); 6,98

(s, 1H, —COOCH< ).

Exemple 6

Une solution de 1 g de benzhydryloxycarbonyl-2 t.butoxycarbonylamino-7 (mésyloxy-2 propène-1 yl-1)-3 oxo-8 thia-5 aza-1 bicyclo[4.2.0] octène-2, forme Z dans 12 cm3 d'acétonitrile est chauffée à 38°C. On ajoute en 20 minutes une solution de 0,63 g de monohydrate d'acide p.toluènesulfonique dans 10 cm3 d'acétonitrile puis on maintient le mélange à 38°C pendant 1 heure. On dilue avec 30 cm3 de dichlorométhane, lave la solution obtenue avec 20 cm3 de solution saturée de bicarbonate de sodium puis avec 50 cm3 d'eau distillée, sèche sur sulfate de sodium et concentre jusqu'à un volume de 15 cm3 sous pression réduite (20 mm de mercure; 2,7 kPa) à 20°C. On obtient ainsi une solution d'amino-7 benzhydryloxycarbonyl-2 (mésyloxy-2 propène-1 yl-1)-3 oxo-8 thia-5 aza-1 bicyclo[4.2.0] octène-2, forme Z dans laquelle on ajoute 0,7 cm3 de triéthylamine (Solution A).

A une suspension de 0,8 g d'acide méthoxyimino-2 (tritylamino-2 thiazolyl-4)-2 acétique, isomère syn dans 12 cm3 de dichlorométhane refroidie à —10°C, on ajoute successivement 0,16 cm3 de N,N-diméthylacétamide et 0,81 cm3 de solution benzénique de phosgène (2,5 M) en 20 minutes. On agite encore pendant 16 heures à —10°C puis on ajoute la solution A goutte à goutte en 25 minutes. On agite la solution pendant 2 heures à —10°C puis on dilue avec 50 cm3 d'acétate d'éthyle et lave avec 50 cm3 d'acide chlorhydrique 0,1 N, 50 cm3 de solution saturée de bicarbonate de sodium puis 50 cm3 d'eau distillée. On sèche sur sulfate de sodium et concentre à sec sous pression réduite (20 mm de mercure; 2,7 kPa) à 20°C. Le résidu est chromatographié sur une colonne de 10 g de gel de silice Merck (0,06—0,2) (diamètre de la colonne: 1,5 cm; hauteur: 12 cm). On élue avec 700 cm3 de mélange acétate d'éthyle/cyclohexane 25—75 (en volumes) en recueillant des fractions de 25 cm3. On concentre à ses les fractions 10 à 21 sous pression réduite (20 mm de mercure; 2,7 kPa) à 20°C. On obtient ainsi 0,43 g de benzhydryloxycarbonyl-2 (mésyloxy-2 propène-1 yl-1)-3 [méthoxyimino-2 (tritylamino-2 thiazolyl-4)-2 acétamido]-7 oxo-8 thia-5 aza-1 bicyclo[4.2.0] octène-2, isomère syn, forme E sous forme d'une meringue jaune.

Spectre de RMN du proton (350 MHz, $CDCl_3$, $\delta$ en ppm, J en Hz) 1,8

(s, =C—$CH_3$);

3,18 (s, —$OSO_2$—$CH_3$); 3,42 et 3,78 (2d, J = 18, —S—$CH_2$—); 4,08 (s, =N—$OCH_3$); 5,13 (d, J = 5, —H en 6); 6,09 (dd, J = 5 et 9, —H en 7); 6,86 (d, J = 9, —CO—NH—); 6,95 (s, —COO—$CH(C_6H_5)_2$); 7,05 (mf, —NH—$C(C_6H_5)_3$); 7,2 à 7,5 (mt aromatiques).

Les produits des exemples suivants peuvent être mis en oeuvre comme décrit aux exemples 1 à 4 pour préparer des produits selon l'invention.

Exemple A

A une solution de 2,06 g de benzhydryloxycarbonyl-2 méthyl-3 phénoxyacétamido-7 oxo-8 thia-5 aza-1 bicyclo[4.2.0] octène-2 dans 15 cm3 de diméthylformamide à 80°C on ajoute en 45 minutes une solution de 1,62 g de diméthylamino-1 méthoxy-1 éthylène dans 3 cm3 de diméthylformamide. Le mélange est ensuite agité pendant 10 minutes à 80°C. On obtient ainsi une solution de benzhydryloxycarbonyl-2 phénoxyacétamido-7 (diméthylamino-2 propène-1 yl-1)-3 oxo-8 thia-5 aza-1 bicyclo[4.2.0] octène-2, qui est versée dans un mélange agité de 100 cm3 d'acétate d'éthyle, 50 cm3 d'eau distillée et 50 cm3 d'acide citrique 1N à 0°C. La phase organique est séparée par décantation, lavée avec 2 fois 100 cm3 d'eau distillée, séchée sur sulfate de sodium et concentrée à sec sous pression réduite (20 mm de mercure; 2,7 kPa) à 30°C. Le résidu est chromatographié sur une colonne de 20 g de gel de silice Merck (0,06—0,2) (diamètre de la colonne: 2 cm; hauteur: 16 cm). On élue avec 600 cm3 de mélange acétate d'éthyle/cyclohexane 28—72 (en volumes) en recueillant des fractions de 30 cm3. Les fractions 7 à 16 sont concentrées à sec sous pression réduite (20 mm de mercure; 2,7 kPa) à 20°C. On obtient ainsi 0,53 g d'acétonyl-3 benzhydryloxycarbonyl-2 phénoxyacétamido-7 oxo-8 thia-5 aza-1 bicyclo[4.2.0] octène-2 sous la forme d'une meringue jaune.

Spectre infra-rouge ($CHCl_3$), bandes caractéristiques ($cm^{-1}$) 3420, 1785, 1725, 1700, 1660, 1520, 1495, 1440, 690.

Spectre de RMN du proton (80 MHz, $CDCl_3$, $\delta$ en ppm, J en Hz) 2,09 (s, 3H, —CO—$CH_3$); 3,10 à 3,90 (mt, 4H, —$CH_2$—CO— et —S—$CH_2$—); 4,57 (s, 2H, —O—$CH_2$—CO—); 5,05 (d, J = 5, 1H, —H en 6); 6,93 (dd, J = 9 et 5, 1H, —H en 7); 6,95 (s et d, J = 8, —COO—$CH(C_6H_5)_2$ et —H aromatiques en ortho du phénoxyméthyle); 7,05 (t, J = 8, —H aromatique en para du phénoxyméthyle); 7,15 à 7,65 (mt, aromatiques et —CO—NH—).

Exemple B

A une solution de 0,56 g d'acétonyl-3 benzhydryloxycarbonyl-2 phénoxyacétamido-7 oxo-8 thia-5 aza-1 bicyclo[4.2.0] octène-2 dans 12 cm3 de dichlorométhane refroidie à −10°C, on ajoute en 25 minutes une solution de 0,2 g d'acide m.chloroperbenzoïque à 85% dans 6 cm3 de dichlorométhane, puis le mélange est maintenu à −10°C pendant 10 minutes. La solution est lavée avec 10 cm3 de solution aqueuse saturée de bicarbonate de sodium, séchée sur sulfate de magnésium puis concentrée à sec sous pression réduite (20 mm de mercure; 2,7 kPa) à 20°C. Le résidu est chromatographié sur une colonne de 10 g de gel de silice Merck (0,06—0,2 (diamètre de la colonne: 1,5 cm; hauteur: 12 cm). On élue avec 600 cm3 de mélange acétate d'éthyle/cyclohexane 50—50 (en volumes) en recueillant des fractions de 10 cm3. On concentre à sec les fractions 26 à 32 sous pression réduite (20 mm de mercure; 2,7 kPa) à 20°C. On obtient ainsi 0,12 g d'acétonyl-3 benzhydryloxycarbonyl-2 phénoxyacétamido-7 oxo-8 oxyde-5 thia-5 aza-1 bicyclo[4.2.0] octène-2 sous la forme d'un solide blanc.

Spectre de RMN du proton (360 MHz, DMSO $d_6$, $\delta$ en ppm, J en Hz) 1,97 (s, 3H, —$CH_3$); 3,54 et 3,81 (2d, J = 16, 2H, —CO—$CH_2$—); 3,70 et 3,87 (2d, J = 18, 2H, —S(O)$CH_2$—); 4,72 (s, 2H, —$OCH_2$—); 5,04 (d, J = 5, 1H, $H_6$); 6,12 (dd, J = 5 et 9, 1H, $H_7$); 6,88

(s, 1H, —COOCH$\langle$ );

8,16 (d, J = 9, —CONH—).

Exemple C

Une suspension constituée par un mélange de 0,21 g de benzhydryloxycarbonyl-2 t.butoxycarbonylamino-7 [$\alpha$-($\beta$-diméthylaminostyryl)]-3 oxo-8 thia-5 aza-1 bicyclo[4.2.0] octène-2, forme E, de 5 cm3 d'acétate d'éthyle et de 3 cm3 d'acide chlorhydrique 1N est agitée fortement à 20°C pendant 2 heures. La phase organique est décantée, lavée par 3 cm3 d'une solution saturée de bicarbonate de sodium, puis par 2 cm3 d'eau, séchée sur sulfate de magnésium et évaporée à sec sous pression réduite (20 mm de mercure; 2,7 kPa) à 20°C. On obtient ainsi 0,195 g de benzhydryloxycarbonyl-2 t.butoxycarbonylamino-7 oxo-8 phénacyl-3 thia-5 aza-1 bicyclo[4.2.0] octène-2 sous forme d'une meringue jaune pâle.

Spectre infra-rouge ($CHBr_3$), bandes caractéristiques ($cm^{-1}$) 3420, 1785, 1720, 1690, 1595, 1580, 1505, 1495, 1455, 1450, 1390, 1370, 760, 745.

Spectre de RMN du proton (35 MHZ, $CDCl_3$, $\delta$ en ppm J en Hz) 1,48 (s, 9H, $(CH_3)_3C$—); 3,32 et 3,64 (2d, J = 19, 2H, —$CH_2S$—); 4,09 et 4,47 (2d, J = 17,2H, —$CH_2CO$—); 5,04 (d, J = 5, 1H, H en 6); 5,31 (d, J = 8, 1H, —CONH—); 5,64 (dd, J = 5 et 8, 1H, H en 7); 6,86

(s, 1H, —COOCH< ).

Exemple D

A une solution de 7,6 g de benzhydryloxycarbonyl-2 t.butoxycarbonylamino-7 méthyl-3 oxo-8 thia-5 aza-1 bicyclo[4.2.0] octène-2 dans 50 cm3 de dioxanne à 80°C, on ajoute en 8 minutes une solution de 7 g de tris-diméthylaminométhylbenzène dans 50 cm3 de dioxanne et on maintient à 80°C pendant 12 minutes sous agitation. A ce stade on obtient un mélange de benzhydryloxycarbonyl-2 t.butoxycarbonylamino-7 [$\alpha$-($\beta$-diméthylaminostyryl)]-3 oxo-8 thia-5 aza-1 bicyclo[4.2.0] octène-2 et octène-3, formes Z et E. Le mélange réactionnel est versé dans un mélange agité de 200 cm3 d'acétate d'éthyle, de 20 cm3 d'acide chlorhydrique 1N et de 20 g de glace. L'agitation est continuée à 20°C pendant 22 heures. La phase organique est séparée, lavée par 50 cm3 d'une solution saturée de bicarbonate de sodium, puis 2 fois par 100 cm3 d'eau distillée, séchée sur sulfate de magnésium et évaporée à sec sous pression réduite (20 mm de mercure; 2,7 kPa) à 20°C. Le résidu (11,2 g) est dissous dans 100 cm3 d'un mélange acétate d'éthyle/cyclohexane 30—70 (en volumes). La solution est alors filtrée sur 50 g de gel de silice Merck (0,06—0,2), lavée par 200 cm3 du même mélange et le filtrat évaporé. On obtient 8,3 g d'un mélange de benzhydrylcarbonyl-2 t.butoxycarbonylamino-7 oxo-8 phénacyl-3 thia-5 aza-1 bicyclo[4.2.0] octène-2 et octène-3 sous forme d'une meringue blonde.

Spectre infra-rouge ($CHBr_3$), bandes caractéristiques ($cm^{-1}$) 3420, 1785, 1720, 1690, 1595, 1580, 1505, 1495, 1455, 1450, 1390, 1370, 760, 745.

Spectre de RMN du proton (350 MHz, $CDCl_3$, $\delta$ en ppm, J en Hz) Mélange 50—50 d'octène-2 et d'octène-3 1,45 (s, 9H, —$C(CH_3)_3$ octène-2 + octène-3); 3,32 et 3,67 (2d, J = 18, 1H, —$SCH_2$— octène-2); 3,69 et 3,92 (2d, J = 17, 1H, —$CH_2CO$— octène-3); 4,08, et 4,46 (2d, J = 17, 1H, —$CH_2CO$— octène-2); 5,07 (d, J = 5, 0,5 H, H en 6 octène-2); 5,24 (d, J = 4, 0,5 H, H en 6 octène-3); 5,30 (s, 0,5 H, H en 2 octène-3); 5,20 à 5,35 (m, 0,5 H —CONH— octène-2); 5,35 à 5,50 (m, 1H, H en 7 et —CONH— octène-3); 5,64 (dd, J = 5 et 9, 0,5 H, —H en 7 octène-2); 6,14 (s, 0,5 H, H en 4 octène-3); 6,86

(s, 1H, —COOCH< octène-2 et octène-3).

Exemple E

A une solution de 4,5 g de benzhydryloxycarbonyl-2 t.butoxycarbonylamino-7 oxo-8 phénacyl-3 thia-5 aza-1 bicyclo[4.2.0] octène-2 et octène-3 dans 90 cm3 de dichlorométhane à 5°C, on ajoute 1,6 g d'acide m.chloroperbenzoïque à 85% et laisse 30 minutes à 5°C sous agitation. Le mélange réactionnel est versé sur 50 cm3 d'une solution saturée de bicarbonate de sodium; la phase organique est lavée par 50 cm3 d'eau distillée, séchée sur sulfate de magnésium et évaporée à sec sous pression réduite (20 mm de mercure; 2,7 kPa) à 20°C. Le résidu (4,25 g) est fixé sur 20 g de gel de silice Merck (0,06—0,2) et chromatographié sur une colonne de 150 g de gel de silice Merck (0,06—0,2) (diamètre de la colonne: 3,6 cm; hauteur: 40 cm). On élue d'abord par 700 cm3 d'un mélange acétate d'éthyle/cyclohexane 80—20 (en volumes) puis par 1 litre d'un mélange acétate d'éthyle/cyclohexane 70—30 (en volumes), par 500 cm3 d'un mélange 65—35, par 500 cm3 d'un mélange 60—40 et enfin par 1 litre d'un mélange 50—50 en recueillant des fractions de 50 cm3. Les fractions 47 à sont réunies et le solvant est évaporé à sec sous pression réduite (20 mm de mercure; 2,7 kPa) à 20°C. On obtient ainsi 2,5 g de benzhydryloxycarbonyl-2 t.butoxycarbonylamino-7 oxo-8 oxyde-5 phénacyl-3 thia-5 aza-1 bicyclo[4.2.0] octène-2 sous forme d'une meringue brune.

Spectra infra-rouge ($CHCl_3$), bandes caractéristiques ($cm^{-1}$) 3410, 1800, 1715, 1685, 1595, 1580, 1500, 1445, 1390, 1370, 1165, 1040.

Spectre de RMN du proton (350 MHz, $CDCl_3$, $\delta$ en ppm, J en Hz) 1,50 (s, 9H, $(CH_3)_3C$—); 3,62 (s, 2H, —$CH_2CO$—); 3,65 et 5,28 (2d, J = 18, 2H, —$CH_2S{\rightarrow}O$); 4,65 (d, J = 4, 1H, H en 6); 5,82 (s large, 2H, H en 7 et —CONH—); 6,86

(s, 1H, —COOCH< ).

Exemple F

A une solution de 8,15 g d'acide p.toluènesulfonique monohydraté dans 170 cm3 d'acétonitrile tiédie à 35°C on ajoute 10,1 g de benzhydryloxycarbonyl-2 t.butoxycarbonylamino-7 oxo-8 oxyde-5 phénacyl-3 thia-5 aza-1 bicyclo[4.2.0] octène-2 et laisse à 35°C sous agitation pendant 1 heure. Le mélange réactionnel est alors versé sur un mélange agité de 150 cm3 d'une solution saturée de bicarbonate de sodium et de 300 cm3 d'acétate d'éthyle. La phase organique est ensuite lavée par 100 cm3 d'eau distillée, séchée sur sulfate de magnésium et évaporée à sec sous pression réduite (20 mm

de mercure; 2,7 kPa) à 20°C. On obtient ainsi 7 g d'amino-7 benzhydryloxycarbonyl-2 oxo-8 oxyde-5 phénacyl-3 thia-5 aza-1 bicyclo[4.2.0] octène-2 brut sous forme d'une huille épaisse utilisable sans purification.

Une solution de 1,25 g d'amino-7 benzhydryloxycarbonyl-2 oxo-8 oxyde-5 phenacyl-3 thia-5 aza-1 bicyclo[4.2.0] octène-2 et de 1,05 cm3 de triéthylamine dans 10 cm3 de dichlorométhane refroidie à 0°C est ajoutée en 1 minute à une solution de chlorure de méthoxy-2 (tritylamino-2 thiazolyl-4)-2 acétyle (préparé à partir de 1,21 g d'acide méthoxyimino-2 (tritylamino-2 thiazolyl-4)-2 acétique, 1,3 cm3 d'une solution 2,4 M de phosgène dans du chlorobenzène, 0,23 cm3 de diméthylacétamide dans 15 cm3 de dichlorométhane pendant 16 heures à −7°C) à −10°C. Le mélange réactionnel est agité, pendant 1 heure à −5°C, puis 1 heure en laissant remonter la température de −5°C à 20°C, puis versé sur 5 cm3 d'acide chlorhydrique 0,5 N. La phase organique est lavée par 10 cm3 d'eau, séchée sur sulfate de magnésium et évaporée à sec sous pression réduite (20 mm de mercure; 2,7 kPa) à 20°C. Le résidu est solidifié dans 20 cm3 d'éther isopropylique. On obtient de cette façon 2 g de benzhydryloxycarbonyl-2 [méthoxyimino-2 (tritylamino-2 thiazolyl-4)-2 acétamido]-7 oxo-8 oxyde-5 phénacyl-3 thia-5 aza-1 bicyclo[4.2.0] octène-2, isomère syn sous forme d'un solide orangé.

Spectre infra-rouge ($CHCl_3$), bandes caractéristiques ($cm^{-1}$) 3400, 1800, 1725, 1680, 1590, 1580, 1510, 1490, 1445, 1040, 690.

Spectre de RMN du proton (350 MHz, $CDCl_3$, $\delta$ en ppm, J en Hz) 3,59 (s large, 2H); 3,68 et 5,22 (2d, J = 18, 2H, $—CH_2S—$ et $—CH_2CO—$); 4,09 (s, 3H, $=NOCH_3$); 4,71 (d, J = 5, 1H, H en 6); 6,16 (dd, J = 5 et 9, 1H, H en 7); 6,74 (s, 1H, H thiazole), 6,85

(s, 1H, —COOCH< );

7,07 (s large, 1H, $—NHC(C_6H_5)_3$); 7.50 (d, J = 9, 1H, —CONH—).

Exemple G

A une solution de 4,3 g de benzhydryloxycarbonyl-2 [méthoxyimino-2 (tritylamino-2 thiazolyl-4)-2 acétamido]-7 oxo-8 oxyde-5 phénacyl-3 thia-5 aza-1 bicyclo[4.2.0] octène-2, isomère syn et de 1,88 cm3 de diméthylacétamide dans 43 cm3 de dichlorométhane refroidie à −8°C, on ajoute en 1 minute 0,81 cm3 de trichlorure de phosphore et laisse pendant 1 heure sous agitation à −8°C. Le mélange est alors versé sur 20 cm3 d'une solution saturée de bicarbonate de sodium et 100 cm3 d'acétate d'éthyle. La phase organique est lavée par 30 cm3 d'eau, séchée sur sulfate de magnésium et évaporée à sec sous pression réduite (20 mm de mercure; 2,7 kPa) à 20°C. Le résidu est dissous dans 15 cm3 d'acétate d'éthyle, la solution est filtrée sur 13 g de gel de silice Merck (0,06—0,2) et lavée par 20 cm3 d'acétate d'éthyle. Le filtrat est évaporé à sec sous pression réduite (20 mm de mercure; 2,7 kPa) à 20°C. On obtient ainsi 3,6 g de benzhydryloxycarbonyl-2 [méthoxyimino-2 (tritylamino-2 thiazolyl-4)-2 acétamido]-7 oxo-8 phénacyl-3 thia-5 aza-1 bicyclo[4.2.0] octène-2, isomère syn sous forme d'un solide beige.

Spectre infra-rouge ($CHCl_3$), bandes caractéristiques ($cm^{-1}$) 3400, 2820, 1780, 1725, 1680, 1590, 1580, 1520, 1490, 1040.

Spectre de RMN du proton (350 MHz, $CDCl_3$, $\delta$ en ppm, J en Hz) 3,30 et 3,63 (2d, J = 18, 2H, $—CH_2S—$); 4,07 (s, 3H, $=NOCH_3$); 4,11 et 4,40 (2d, J = 18, 2H, $—CH_2CO—$); 5,11 (d, J = 5, 1H, H en 6); 5,95 (dd, J = 5 et 9, 1H, H en 7); 6,77 (s, 1H, H du thiazole); 6,87

(s, 1H, —COOCH< );

6,91 (d, J = 9, 1H, —CONH—); 7,03 (s, 1H, $—NHC(C_6H_5)_3$).

Exemple H

A une solution de 3,75 g de t.butoxycarbonylamino-7 (méthoxy-4 benzyloxycarbonyl-2 méthyl-3 oxo-8 thia-5 aza-1 bicyclo[4.2.0] octène-2 dans 25 cm3 de diméthylformamide portée à 80°C on ajoute en 55 minutes 4 cm3 de diméthylamino-1 méthoxy-1 éthylène en maintenant la température à 80°C. On obtient ainsi une solution de t.butoxycarbonylamino-7 (diméthylamino-2 propène-1 yl-1)-3 (méthoxy-4 benzyloxycarbonyl)-2 oxo-8 thia-5 aza-1 bicyclo[4.2.0] octène-2 que l'on verse dans un mélange de 200 cm3 d'eau glacée et 150 cm3 d'acétate d'éthyle. La phase organique est lavée par 2 fois 200 cm3 d'eau, séchée sur sulfate de sodium, filtrée en présence de noir décolorant et évaporée à sec sous pression réduite (20 mm de mercure; 2,7 kPa). Le résidu est chromatographié sur une colonne de 45 g de gel de silice Merck (0,06—0,2) (diamètre de la colonne: 3 cm, hauteur: 15 cm). On élue avec 500 cm3 d'un mélange acétate d'éthyle/cyclohexane 20—80 (en volumes) et recueille des fractions de 40 cm3. Les fractions 7 à 10 sont concentrées à sec sous pression réduite (20 mm de mercure; 2,7 kPa) et on obtient ainsi 0,25 g d'un mélange d'acétonyl-3 t.butoxycarbonylamino-7

(méthoxy-4 benzyloxycarbonyl)-2 oxo-8 thia-5 aza-1 bicyclo[4.2.0] octène-2 et octène-3 (70—30) sous forme d'un solide ocre.

Rf = 0,27 Plaque de silice Merck $F_{254}$, épaisseur 0,25 mm, éluant: acétate d'éthyle/cyclohexane 40—60 (en volumes).

Spectre de masse: M = 476.

Spectre de RMN du proton (350 MHz, $CDCl_3$, $\delta$ en ppm, J en Hz) 1,47 (s, 9H, —$C(CH_3)_3$ de l'octène-2 et de l'octène-3); 2,03 (s, —CO—$CH_3$ de l'octène-3); 2,12 (s, —CO—$CH_3$ de l'octène-2); 3,18 et 3,33 (2d, J = 17,5, —$CH_2$—CO— de l'octène-3); 3,30 et 3,55 (2d, J = 18, —$CH_2$—S— de l'octène-2); 3,51 et 3,73 (2d, J = 17,5, —$CH_2$—CO— de l'octène-2); 3,80 (s, —$OCH_3$ de l'octène-2); 3,82 (s, —$OCH_3$ de l'octène-3); 4,95 (d, J = 4,5 —H en 6 de l'octène-2 et s, —H en 2 de l'octène-3; 5,07 (s, —COO—$CH_2$— de l'octène-3); 5,13 et 5,20 (2d, J = 12, —COO—$CH_2$— de l'octène-2); 5,21 (d, J = 4, —H en 6 de l'octène-3); 5,33 (d, J = 9, —CO—NH— de l'octène-2); 5,42 (mf, —CO—NH— et —H en 7 de l'octène-3); 5,59 (dd, J = 9 et 4,5, —H en 7 de l'octène-2); 6,06 (s, —H en 4 de l'octène-3); 6,89 (mt, —H aromatiques en ortho du —$OCH_3$ de l'octène-2 et de l'octène-3); 7,28 (d, J = 7,5, —H aromatiques en méta du —$OCH_3$ de l'octéne-3); 7,34 (d, J = 7,5, —H aromatiques en ortho du —$OCH_3$ de l'octène-2).

Exemple I

A une solution de 5,38 g d'un mélange d'environ 50/50 de benzhydryloxycarbonyl-2 méthyl-3 oxo-8 tritylamino-7 thia-5 aza-1 bicyclo[4.2.0] octène-2 et octène-3 dans 25 cm3 de diméthylformamide portée à 80°C, on ajoute, en 56 minutes, en maintenant la température à 80°C, 4 cm3 de diméthylamino-1 méthoxy-1 éthylène. On obtient ainsi une solution d'un mélange de benzhydryloxycarbonyl-2 (diméthylamino-2 propène-1 yl-1)-3- oxo-8 tritylamino-7 thia-5 aza-1 bicyclo[4.2.0] octène-2 et octène-3 que l'on verse dans un mélange de 200 cm3 d'eau glacée et 200 cm3 d'acétate d'éthyle. La phase organique est lavée par 2 fois 200 cm3 d'eau, séchée sur sulfate de sodium, filtrée en présence de noir décolorant et évaporée à sec sous pression réduite (20 mm de mercure; 2,7 kPa). Le résidu est chromatographié sur une colonne de 60 g de gel de silice Merck (0,06—0,2) (diamètre de la colonne: 3 cm, hauteur: 20 cm). On élue avec 1000 cm3 d'un mélange acétate d'éthyle/cyclohexane 15—85 (en volumes) en recueillant des fractions de 60 cm3. Les fractions 9 à 15 sont réunies et évaporés à sec sous pression réduite (20 mm de mercure; 2,7 kPa). On obtient ainsi 0,51 g d'un mélange environ 50/50 d'acétonyl-3 benzhydryloxycarbonyl-2 oxo-8 tritylamino-7 thia-5 aza-1 bicyclo[4.2.0] octène-2 et 3 sous forme d'un solide jaune.

Rf = 0,18 Plaque de silice Merck $F_{254}$, épaisseur 0,25 mm; éluant: acétate d'éthyle/cyclohexane 20—80 (en volumes).

Spectre de masse M = 664.

Spectre de RMN du proton (350 MHz, $CDCl_3$, $\delta$ en ppm, J en Hz) 1,88 (s, —CO—$CH_3$ de l'octène-3); 2 (s, —CO—$CH_3$ de l'octène-2); 3 et 3,18 (2d, J = 16, —$CH_2$—CO— de l'octène-3); 3,02

(d, J = 11, NH de l'octène-3);

3,11 et 3,34 (2d, J = 18, —$CH_2$—S— de l'octéne-2); 3,14

(d, J = 8, NH de l'octène-2);

3,44 et 3,66 (2d, J = 17, —$CH_2$—CO— de l'octène-2); 4,23 (d, J = 3,5, —H en 6 de l'octène-3); 4,31 (d, J = 4,5, —H en 6 de l'octène-2); 4,58 (dd, J = 3,5 et 11, —H en 7 de l'octène-3); 4,72 (dd, J = 8 et 4,5, —H en 7 de l'octène-2); 5 (s, —H en 2 de l'octène-3); 5,89 (s, —H en 4 de l'octène-3); 6,70 (s, —$COOCH(C_6H_5)_2$ de l'octène-3); 6,85 (s, —$COOCH(C_6H_5)_2$ de l'octène-2); 7,15 à 7,60 (mt, 25H aromatiques).

Exemple J

A une solution de 2,58 g de benzhydryloxycarbonyl-2 diéthoxyphosphoramido-7 méthyl-3 oxo-8 thia-5 aza-1 bicyclo[4.2.0] octène-2 dans 10 cm3 de diméthylformamide portée à 80°C, on ajoute, en 55 minutes en maintenant la température à 80°C, une solution de 2,3 cm3 de diméthylamino-1 méthoxy-1 éthylène dans 4 cm3 de diméthylformamide. On obtient ainsi une solution de benzhydryloxycarbonyl-2 diéthoxyphosphoramido-7 (diméthylamino-2 propène-1 yl-1)-3 oxo-8 thia-5 aza-1 bicyclo[4.2.0] octène-2 que l'on verse dans un mélange de 150 cm3 d'eau glacée et 100 cm3 d'acétate d'éthyle. La phase organique est lavée par 250 cm3 d'eau, séchée sur sulfate de sodium, filtrée en présence de noir décolorant et évaporée à sec sous pression réduite (20 mm de mercure; 2,7 kPa). Le résidu est chromatographié sur une colonne de 30 g de gel de silice Merck (0,06—0,2) (diamètre de la colonne: 2,2 cm, hauteur: 18 cm). On élue avec 100 cm3 d'un mélange acétate

d'éthyle/cyclohexane 50—50 (en volumes) puis 300 cm3 d'un mélange acétate d'éthyle/cyclohexane 70—30 (en volumes) en recueillant des fractions de 30 cm3. Les fractions 9 à 12 sont réunies et évaporées à sec sous pression réduite (20 mm de mercure; 2,7 kPa). On obtient ainsi 0,31 g d'un mélange 75/25 d'acétonyl-3 benzhydryloxycarbonyl-2 diéthoxyphosphoramido-7 oxo-8 thia-5 aza-1 bicyclo[4.2.0] octène-2 et octène-3 sous forme d'un solide jaune.

Rf = 0,36 [plaque de silice Merck $F_{254}$, épaisseur 0,25 mm; éluant: acétate d'éthyle/cyclohexane 80—20 (en volumes)].

Spectre de RMN du proton (350 MHz, DMSO, $d_6$, $\delta$ en ppm, J en Hz) 1,15 à 1,30 (mt, 6H, —$CH_3$ de l'octène-2 et de l'octène-3); 1,88 (s, —CO—$CH_3$ de l'octène-3); 1,96 (s, —CO—$CH_3$ de l'octène-2); 3,24 et 3,44 (2d, J = 17,5, —$CH_2$—CO— de l'octène-3); 3,40 et 3,60 (2d, J = 18,5, —$CH_2$—S— de l'octène-2); 3,53 et 3,65 (2d, J = 17,5, —$CH_2$—CO— de l'octène-2); 3,90 à 4,05 (mt, 4H, —$OCH_2$— de l'octène-2 et de l'octène-3); 4,97 (dt, J = 11 et 4,5, —H en 7 de l'octène-3); 5,06 (d, J = 4,5, —H en 6 de l'octène-3); 5,08 (d, J = 4,5, —H en 6 de l'octène-2); 5,11 (s large, —H en 2 de l'octène-3); 5,16 (dt, J = 11 et 4,5, —H en 7 de l'octène-2); 6,24

(t, J = 11, >N—H de l'octène-2);

6,27 (t, J = 11, >N—H de l'octène-3);

6,40 (s large, —H en 4 de l'octène-3); 6,77 (s, —COOC*H*$(C_6H_5)_2$ de l'octène-3); 6,84 (s, —COO—C*H*$(C_6H_5)_2$ de l'octène-2); 7,25 à 7,55 (mt, 10H, aromatique de l'octène-2 et de l'octène-3).

L'exemple suivant montre comment les produits de formule générale (I) peuvent être utilisés.

Exemple De Reference

Le produit de l'exemple 1 peut être utilisé de la manière suivante:

On ajoute 1,53 g de sel de sodium de mercapto-5 méthyle-2 thiadiazole-1,3,4 à une solution de 5,9 g de mélange 66—34 (en moles) de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z dans 100 cm3 de diméthylformamide à 20°C. On agite pendant 1 heure 10 minutes à 20°C puis dilue la solution avec 500 cm3 d'eau. On extrait avec 300 cm3 d'acétate d'éthyle, sèche la phase organique sur sulfate de sodium, concentre à sec à 20°C sous pression réduite (20 mm de mercure; 2,7 kPa) et chromatographie le résidu sur une colonne de 100 g de gel de silice Merck (0,06—0,2) (diamètre de la colonne: 3,5 cm; hauteur: 25 cm). On élue avec 1 litre de mélange acétate d'éthyle/cyclohexane 35—65 (volumes) et recueille des fractions de 60 cm3. On concentre à sec à 20°C les fractions 8 à 14 sous pression réduite (20 mm de mercure; 2,7 kPa). On obtient ainsi 1,35 g de mélange 40—60 de benzhydryoxycarbonyl-2 t.butoxycarbonylamino-7 {[(méthyl-2 thiadiazol-1,3,4 yl-5) thio]-2 propène-1 yl-1}-3 oxo-8 thia-5 aza-1 bicyclo[4.2.0] octène-2 et octène-3, forme Z sous forme d'une meringue jaune.

Spectre infra-rouge ($CHCl_3$), bandes caractéristiques ($cm^{-1}$) 3440, 1780, 1720, 1505, 1455, 1390, 1370, 1160.

Spectre de RMN du proton (350 MHz, $CDCl_3$, $\delta$ en ppm, J en Hz) 1,48 (s, 9H, —$C(CH_3)_3$); 2,06

(s large, $CH_3$—C=, octène-2 et -3);

2,74 (s, $CH_3$—Het de l'octène-2); 2,76 (s, $CH_3$—Het de l'octène-3); 3,66 et 3,76 (2d, J = 18, —$SCH_2$— octène-3); 5,04 (d, J = 4, $H_6$ octène-2); 5,19 (s, $H_2$ octène-3); 5,25 (d, J = 4, $H_6$ octène-3); 5,31 (d, J = 8, —CONH— octène-2); 5,36 à 5,46 (m, $H_7$ et —CONH— octène-3); 5,66 (dd, J = 4 et 8, $H_7$ octène-2); 6,18 (s, $H_4$ octène-3); 6,58

(s, —CH=C— octène-3);

6,64 (s, —CH=C— octène-2);

6,90 (s, —COOCH< octène-3);

6,97 (s, —COOCH< octène-2).

On refroidit à 0°C une solution de 0,22 g de benzhydryloxycarbonyl-2 t.butoxycarbonylamino-7 {[(méthyl-2 thiadiazol-1,3,4 yl-5) thio]-2 propène-1 yl-1}-3 oxo-8 thia-5 aza-1 bicyclo[4.2.0] octène-2 et octène-3, forme Z dans 10 cm3 de dichlorométhane. On ajoute goutte à goutte en 20 minutes une solution de 0,07 g d'acide m.chloroperbenzoïque à 85% dans 5 cm3 de dichlorométhane. On agite ensuite la solution pendant 5 minutes à 0°C, lave avec 20 cm3 de solution saturée de bicarbonate de sodium, sèche sur sulfate de sodium et concentre à sec sous pression réduite (20 mm de mercure; 2,7 kPa) à 20°C. Le résidu est chromatographié sur une colonne de 4 g de gel de silice Merck (0,06—0,2) (diamètre de la colonne: 1 cm; hauteur: 10 cm). On élue avec 100 cm3 de mélange acétate d'éthyle/cyclohexane 60—40 (volumes) et recueille des fractions de 3 cm3. On concentre à sec sous pression réduite (20 mm de mercure; 2,7 kPa) à 20°C les fractions 11 à 22. On obtient ainsi 0,12 g de benzhydryloxycarbonyl-2 t.butoxycarbonylamino-7 {[(méthyl-2 thiadiazol -1,3,4 yl-5) thio]-2 propène-1 yl-1}-3 oxo-8 oxyde-5 thia-5 aza-1 bicyclo[4.2.0] octène-2, forme Z sous forme d'une meringue blanche.

Spectre infra-rouge ($CHCl_3$), bandes caractéristiques ($cm^{-1}$) 3420, 1800, 1720, 1505, 1455, 1370, 1160, 1045, 695.

Spectre de RMN du proton (350 MHz, $CDCl_3$, $\delta$ en ppm, J en Hz) 1,48 (s, 9H, —$C(CH_3)_3$); 2,05 (s, 3H, —$CH_3$); 2,72 (s, 3H, $CH_3$— Hétérocycle); 3,70 et 4,03 (2d, J = 18, 2H, —$SCH_2$—); 4,58 (d, J = 4, 1H, $H_6$); 5,77 (d, J = 8, 1H, —CONH—); 5,85 (dd, J = 4 et 9, 1H, $H_7$); 6,75 (s, 1H, —CH=); 6,98

(s, —COOCH< ).

On chauffe à 40°C une solution de 4,45 g de benzhydryloxycarbonyl-2 t.butoxycarbonylamino-7 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z dans 150 cm3 d'acétonitrile. On ajoute goutte à goutte en 5 minutes une solution de 2,59 g d'acide p.toluènesulfonique (monohydraté) dans 75 cm3 d'acétonitrile. Le mélange est agité à 40°C pendant 35 minutes puis versé dans 200 cm3 de solution aqueuse saturée de bicarbonate de sodium. On ajoute 500 cm3 d'eau distillée et extrait avec 500 cm3 de dichlorométhane. La phase organique est lavée avec 500 cm3 d'eau distillée, séchée sur sulfate de magnésium et concentrée à sec sous pression réduite (20 mm de mercure; 2,7 kPa) à 20°C. On obtient ainsi 3,53 g d'amino-7 benzhydryloxycarbonyl-2 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z sous la forme d'une meringue brune.

Spectre infra-rouge ($CHCl_3$), bandes caractéristiques ($cm^{-1}$) 3420, 1800, 1725, 1500, 1455, 1370, 1160, 1050, 695.

On refroidit à 4°C une solution de 3,53 g d'amino-7 benzhydryloxycarbonyl-2 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z dans 250 cm3 de dichlorométhane. On ajoute successivement 3,28 g d'acide méthoxyimino-2 (tritylamino-2 thiazolyl-4)-2 acétique et 1,54 g de dicyclohexylcarbodiimide. On agite de mélange pendant 1 heure 1/2 à 4°C puis concentre à sec sous pression réduite (20 mm de mercure; 2,7 kPa) à 20°C. Le résidu est repris avec 50 cm3 d'acétate d'éthyle et l'insoluble est séparé par filtration. La solution est lavée avec 100 cm3 de solution aqueuse saturée de bicarbonate de sodium puis avec 100 cm3 d'acide chlorhydrique 0,1N. On sèche sur sulfate de sodium et concentre à sec sous pression réduite (20 mm de mercure; 2,7 kPa) à 20°C. Le résidu est chromatographié sur une colonne de 80 g de gel de silice Merck (0,06—0,2) (diamètre de la colonne: 3 cm; hauteur: 64 cm). On élue avec 300 cm3 d'un mélange acétate d'éthyle/cyclohexane 35—65 (en volumes) puis avec 1200 cm3 du mélange acétate d'éthyle/cyclohexane 70—30 (en volumes) en recueillant des fractions de 60 cm3. On concentre à sec les fractions 12 à 20 sous pression réduite (20 mm de mercure; 2,7 kPa) à 20°C. On obtient ainsi 2 g de benzhydryloxycarbonyl-2 {[(méthyl-2 thiadiazol -1,3,4 yl-5) thio]-2 propène-1 yl-1}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme Z sous la forme d'une meringue blanche.

Spectre infra-rouge (KBr), bandes caractéristiques ($cm^{-1}$) 3420, 1800, 1730, 1685, 1530, 1495, 1450, 1040, 755, 705.

Spectre de RMN du proton (350 MHz, $CDCl_3$, $\delta$ en ppm, J en Hz) 2,05

(s, 3H, $CH_3$—C=);

2,72 (s, 3H, $CH_3$— hétérocycle); 3,71 et 4,06 (2d, J = 18, 2H, —$SCH_2$—); 4,12 (s, 3H, =$NOCH_3$); 4,61 (d, J = 5, 1H, $H_6$); 6,21 (dd, J = 5 et 9, 1H, $H_7$); 6,73 (s, 1H, —CH=); 6,76 (s, 1H, H sur thiazole); 6,97

(s, 1H, —COOCH );

7,14 (s, large, 1H, —NH trityle); 7,44 (d, J = 9, 1H, —CONH—).

Une solution de 2,52 g de benzhydryloxycarbonyl-2 {[(méthyl-2 thiadiazol-1,3,4 yl-5) thio]-2 propène-1 yl-1}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme Z dans 40 cm3 de dichlorométhane est refroidie à −10°C. On ajoute successivement 0,95 cm3 de N,N-diméthylacétamide et 0,7 g de trichlorure de phosphore. La solution est agitée pendant 20 minutes à −10°C puis versée dans 50 cm3 de solution aqueuse saturée de bicarbonate de sodium. On dilue avec 200 cm3 d'eau distillée et extrait avec 300 cm3 d'acétate d'éthyle. La phase organique est séchée sur sulfate de sodium et concentrée à sec sous pression réduite (20 mm de mercure; 2,7 kPa) à 20°C. Le résidu est chromatographié sur une colonne de 25 g de gel de silice Merck (0,06—0,20) (diamètre de la colonne: 2 cm; hauteur: 15 cm). On élue avec 500 cm3 de mélange acétate d'éthyle/cyclohexane 50—50 (en volumes) en recueillant des fractions de 30 cm3. On concentre à sec les fractions 4 à 13 sous pression réduite (20 mm de mercure; 2,7 kPa) à 20°C. On obtient ainsi 0,77 g de benzhydryloxycarbonyl-2 {[(méthyl-2 thiadiazol -1,3,4 yl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z sous la forme d'une meringue blanche.

Spectre infra-rouge (KBr), bandes caractéristiques ($cm^{-1}$) 1790, 1725, 1685, 1525, 1495, 1450, 1050, 750, 700.

Spectre de RMN du proton (350 MHz, $CDCl_3$, $\delta$ en ppm, J en Hz) 2,02

(s, 3H, $CH_3$—C=);

2,7 (s, 3H, $CH_3$— hétérocycle); 3,63 et 3,74 (2d, J = 18, 2H, —$SCH_2$—); 4,07 (s, 3H, =$NOCH_3$); 5,09 (d, J = 4, 1H, $H_6$); 5,94 (dd, J = 4 et 9, 1H, $H_7$); 6,6 (s, 1H, —CH=); 6,76 (s, 1H, H thiazole); 6,84 (d, J = 9, 1H, —CONH—); 6,94

(s, 1H, —COOCH );

7,04 (s large, 1H, —NH— trityle).

On dissout 0,76 g de benzhydryloxycarbonyl-2 {[(méthyl-2 thiadiazol-1,3,4 yl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z dans 8 cm3 d'acide formique. On chauffe la solution à 50°C et ajoute goutte à goutte 4 cm3 d'eau distillée en 10 minutes. On agite pendant 30 minutes à 50°C puis ajoute 4 cm3 d'eau distillée, refroidit, filtre et concentre la solution à sec sous pression réduite (1 mm de mercure; 0,13 kPa) à 40°C. On ajoute 40 cm3 d'éthanol qu'on évapore sous pression réduite (1 mm de mercure; 0,13 kPa) à 30°C et on répète encore 2 fois cette opération. On reprend le résidu avec 10 cm3 d'éthanol, filtre, lave le gâteau avec 20 cm3 d'éther diéthylique, sèche sur sulfate de sodium et évapore le solvant sous pression réduite (1 mm de mercure; 0,13 kPa) à 20°C. On isole ainsi 60 mg d'[(amino-2 thiazolyl-4)-2 méthoxyimino-2 acétamido]-7 carboxy-2 {[(méthyl-2 thiadiazol -1,3,4 yl-5) thio]-2 propène yl-1}-3 oxo-8 thia-5 aza-1 bicyclo[4.2.0] octène-2, isomère syn, forme Z sous forme d'une poudre jaune.

Spectre infra-rouge (KBr), bandes caractéristiques ($cm^{-1}$) 3600, 2700, 1765, 1670, 1625, 1535, 1365, 1035.

Spectre de RMN du proton (350 MHz, DMSO $d_6$, $\delta$ en ppm, J en Hz) 2,24

(s, large, =C—$CH_3$);

2,75 (s, —$CH_3$ thiadiazole); 3,87 (s, =N—$OCH_3$); 4,51 (d, J = 14, 1H des —H du —S—$CH_2$—); 5,44 (dd, J = 5 et 9, —H en 7); 5,62 (d, J = 5, —H en 6); 6,75 (s, —H thiazole); 7,07 (s, —CH=); 7,20 (s large, —$NH_2$); 9,55 (d, J = 9, —CO—NH—).

Les produits des exemples 2, 3, 5 et 6 peuvent être utilisés pour préparer un produit de formule générale (XVIII) par analogie avec la méthode dans l'exemple de référence ci-dessus.

**Revendications pour les Etats contractants: BE CH DE FR GB IT LI LU NL SE**

1. Un nouveau dérivé de la céphalosporine de formule générale:

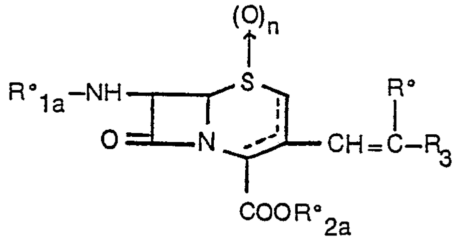

(I)

dans laquelle

le symbole $R^\circ_{1a}$ représente:

a) un radical de formule générale:

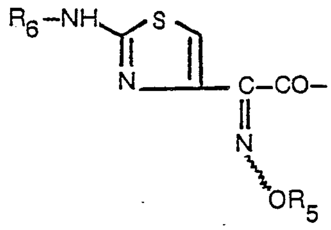

(II)

[dans laquelle $R_5$ est un atome d'hydrogène, un radical alcoyle, vinyle ou cyanométhyle, un groupement protecteur d'oxime ou un radical de formule générale:

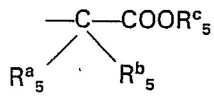

(III)

(dans laquelle $R^a_5$ et $R^b_5$ qui sont identiques ou différents sont des atomes d'hydrogène, des radicaux alcoyle, ou forment ensemble un radical alcoylène contenant 2 ou 3 atomes de carbone, et $R^c_5$ est un atome d'hydrogène ou un radical protecteur d'acide) et $R_6$ est un atome d'hydrogène ou un radical protecteur d'amino], ou

b) un atome d'hydrogène, ou

c) un radical benzhydryle, trityle, un radical acyle de formule générale:

$$R_7CO—\qquad (IVa)$$

[dans laquelle $R_7$ est un radical alcoyle substitué par un radical phényle ou phénoxy], un radical de formule générale:

$$R_8OCO—\qquad (IVb)$$

[dans laquelle $R_8$ est un radical alcoyle ramifié non substitué ou alcoyle droit ou ramifié portant un ou plusieurs substituants [choisis parmi phényle et phényle substitué (par un radical alcoyloxy, nitro ou phényle)], ou vinyle], un radical de formule générale:

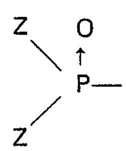

(IVc)

dans laquelle les symboles Z représentent des radicaux phényle ou —OZ' dans lequel Z' représente un radical alcoyle, trichloro-2,2,2 éthyle, phényle ou benzyle (ces 2 derniers radicaux pouvant être substitués par un atome d'halogène ou un radical alcoyle, alcoyloxy ou nitro), ou bien les symboles Z' des 2 substituants Z forment ensemble un radical alcoylène contenant 2 ou 3 atomes de carbone, ou bien $R^\circ_{1a}NH$ est remplacé par un radical méthylèneamino dans lequel le radical méthylène est substitué par un groupement dialcoylamino ou aryle (lui-même éventuellement substitué par 1 ou plusieurs radicaux méthoxy ou nitro),

le symbole $R^o_{2a}$ représente un atome d'hydrogène ou un radical protecteur d'acide

le symbole $R^o$ représente un radical d'un premier groupe constitué par un radical phényle éventuellement substitué [par un radical alcoyle, trifluorométhyle, dialcoylaminométhyle, alcoyloxy, alcoylthio, dialcoylamino ou par un atome d'halogène tel que le fluor ou le chlore], un radical hétérocyclyle à 5 ou 6 chaînons contenant 1 seul hétéroatome tel que pyridyle-2 ou -3, thienyle-2 ou -3 furyle -2 ou -3 éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons, ou un radical hétérocyclyle à 5 ou 6 chaînons contenant un atome d'oxygène ou de soufre et

le symbole $R_3$ représente un radical de formule générale:

$$R_4SO_2O— \qquad (V)$$

dans laquelle $R_4$ représente un radical alcoyle, trihalogénométhyle, ou phényle éventuellement substitué par un atome d'halogène ou par un ou plusieurs radicaux alcoyle ou nitro, étant entendu que lorsque $n = 0$, le produit se présente sous forme bicyclooctène-2 ou -3 et lorsque $n = 1$, le produit se présente sous forme bicyclooctène-2 et que les portions ou radicaux alcoyles sont (sauf mention spéciale) droits ou ramifiés et contiennent 1 à 4 atomes de carbone.

2. Un dérivé de la céphalosporine selon la revendication 1 caractérisé en ce que $R^o_{1a}$ représente un radical de formule générale:

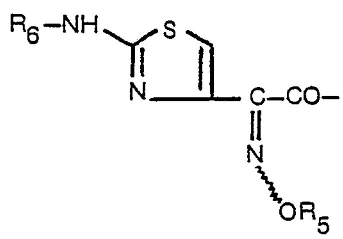

(II)

dans laquelle $R_6$ est défini comme dans la revendication 1 et $R_5$ est un atome d'hydrogène, un radical alcoyle, vinyle, cyanométhyle, trityle, tétrahydropyrannyle ou méthoxy-2 propyle-2, ou un radical de formule générale:

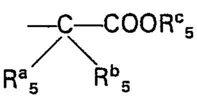

(III)

dans laquelle $R^a_5$ et $R^b_5$ sont définis comme dans la revendication 1 et $R^c_5$ est un atome d'hydrogène ou un radical méthoxyméthyle, t.butyle, benzhydryle, benzyle, nitrobenzyle ou p.méthoxybenzyle.

3. Un dérivé de la céphalosporine selon la revendication 1 caractérisé en ce que $R^o_{2a}$ est un atome d'hydrogène ou un radical protecteur choisi parmi méthoxyméthyle, t.butyle, benzhydryle, benzyle, nitrobenzyle ou p.méthoxybenzyle.

4. Un dérivé de la céphalosporine selon la revendication 1 caractérisé en ce que

$n = 0$ ou 1,

le symbole $R^o_{1a}$ est un atome d'hydrogène ou un radical de formule générale $R_8OCO—$ dans laquelle

$R_8$ est défini comme dans la revendication 1

le symbole $R^o_{2a}$ est un radical protecteur

le symbole $R^o$ est un radical alcoyle, et

le symbole $R_3$ est un radical de formule générale $R_4SO_2O—$ dans laquelle $R_4$ est un radical alcoyle, trihalogénométhyle, ou phényle éventuellement substitué par un radical alcoyle.

5. Un dérivé de la céphalosporine selon la revendication 1, caractérisé en ce que

$n = 0$ ou 1,

$R^o_{1a}$ est un atome d'hydrogène ou un radical de formule générale $R_8OCO—$ dans laquelle $R_8$ est un radical alcoyle ramifié,

$R^o_{2a}$ est un radical benzhydryle,

$R^o$ est un radical méthyle et

$R_3$ est un radical de formule générale $R_4SO_2O—$ dans laquelle $R_4$ est alcoyle, trifluorométhyle ou phényle substitué par un radical méthyle.

6. Un procédé de préparation d'un produit selon la revendication 1 pour lequel $R^o_{1a}$ est défini comme dans la revendication 1 en a) ou c) et $R^o_{2a}$, $R_3$ et n sont définis comme dans la revendication 1, caractérisé en ce que l'on fait agir une forme activée d'un acide $R_4SO_3H$, de formule générale:

$(R_4SO_2)_2O$ (VIa)

ou $R_4SO_2Hal$ (VIb)

dans laquelle $R_4$ est défini comme dans la revendication 1 et Hal représente un atome d'halogène, sur une cétone de formule générale:

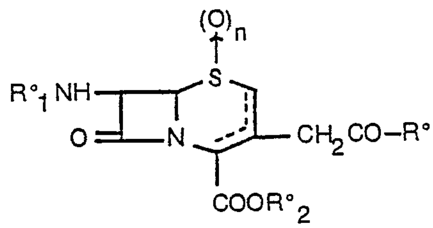

(VII)

[dans laquelle $R°_1$ est défini comme $R°_{1a}$ ci-dessus, à l'exception pour $R_6$ de représenter un atome d'hydrogène lorsque $R°_1$ répond à la définition donnée en a) dans la revendication 1, $R°_2$ est défini comme $R°_{2a}$ et R° et n sont définis comme dans la revendication 1, et qui se présente sous forme bicyclooctène-2 ou -3 lorsque n = 0 et sous forme bicyclooctène-2 lorsque n = 1], ou sur un mélange de ses isomères, puis on réduit éventuellement le sulfoxyde obtenu lorsque n = 1, élimine éventuellement les radicaux protecteurs, et sépare éventuellement les isomères obtenus.

7. Un procédé de préparation d'un produit selon la revendication 1, pour lequel $R°_{1a}$ est un atome d'hydrogène et $R°_{2a}$, $R_3$ et n sont définis comme dans la revendication 1, caractérisé en ce que l'on élimine le radical $R°_{1a}$ ou élimine simultanément les radicaux protecteurs $R°_{1a}$ et $R°_{2a}$ d'un produit selon la revendication 1 pour lequel $R°_{1a}$ est défini comme dans la revendication 1 en c) et $R°_{2a}$, $R_3$ et n sont définis comme dans la revendication 1, puis on sépare éventuellement les isomères obtenus.

8. Un procédé de préparation d'un produit selon la revendication 1 pour lequel $R°_{1a}$ est défini comme dans la revendication 1 en a) et $R°_{2a}$, $R_3$ et n sont définis comme dans la revendication 1, caractérisé en ce que l'on acyle une céphalosporine selon la revendication 1 pour laquelle $R°_{1a}$ est un atome d'hydrogène, ou un mélange de ses isomères, au moyen d'un acide de formule générale:

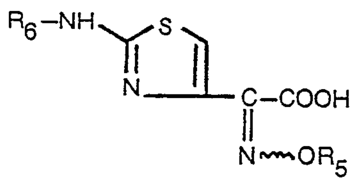

(X)

[dans laquelle $R_5$ et $R_6$ sont définis comme dans la revendication 1 en a) à l'exception pour $R_6$ et pour $R^c_5$ (dans $R_5$) de représenter un atome d'hydrogène] ou d'un dérivé réactif de cet acide, puis on réduit éventuellement le sulfoxyde obtenu, élimine éventuellement les groupements protecteurs et sépare éventuellement les isomères obtenus.

9. Un procédé de préparation d'un produit selon la revendication 1 pour lequel n égale 1, caractérisé en ce que l'on oxyde un produit selon la revendication 1 pour lequel $R°_{1a}$ est défini comme ci-dessus et n égale 0, puis on sépare éventuellement les isomères obtenus.

**Revendication pour l'Etat contactant: AT**

1. Procédé de préparation d'un nouveau dérivé de la céphalosporine de formule générale:

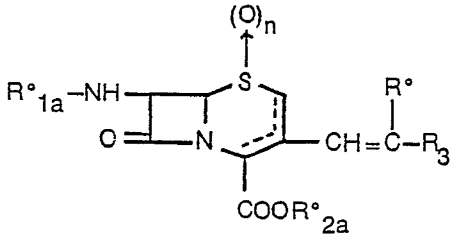

(I)

dans laquelle

le symbole $R°_{1a}$ représente:

a) un radical de formule générale:

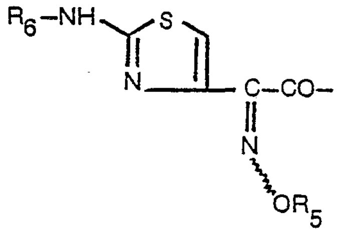

(II)

[dans laquelle $R_5$ est un atome d'hydrogène, un radical alcoyle, vinyle ou cyanométhyle, un groupement protecteur d'oxime ou un radical de formule générale:

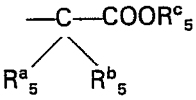

(III)

(dans laquelle $R^a_5$ et $R^b_5$ qui sont identiques ou différents sont des atomes d'hydrogène, des radicaux alcoyle, ou forment ensemble un radical alcoylène contenant 2 ou 3 atomes de carbone, et $R^c_5$ est un atome d'hydrogène ou un radical protecteur d'acide) et $R_6$ est un atome d'hydrogène ou un radical protecteur d'amino], ou

b) un atome d'hydrogène, ou

c) un radical benzhydryle, trityle, un radical acyle de formule générale:

$$R_7CO— \qquad (IVa)$$

[dans laquelle $R_7$ est un radical alcoyle substitué par un radical phényle ou phénoxy], un radical de formule générale:

$$R_8OCO— \qquad (IVb)$$

[dans laquelle $R_8$ est un radical alcoyle ramifié non substitué ou alcoyle droit ou ramifié portant un ou plusieurs substituants [choisis parmi phényle et phényle substitué (par un radical alcoyloxy, nitro ou phényle)], ou vinyle], un radical de formule générale:

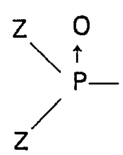

(IVc)

dans laquelle les symboles Z représentent des radicaux phényle ou —OZ' dans lequel Z' représente un radical alcoyle, trichloro-2,2,2 éthyle, phényle ou benzyle (ces 2 derniers radicaux pouvant être substitués par un atome d'halogène ou un radical alcoyle, alcoyloxy ou nitro), ou bien les symboles Z' des 2 substituants Z forment ensemble un radical alcoylène contenant 2 ou 3 atomes de carbone, ou bien $R°_{1a}NH$ est remplacé par un radical méthylèneamino dans lequel le radical méthylène est substitue par un groupement dialcoylamino ou aryle (lui-même éventuellement substitué par 1 ou plusieurs radicaux méthoxy ou nitro),

le symbole $R°_{2a}$ représente un atome d'hydrogène ou un radical protecteur d'acide

le symbole R° représente un radical d'un premier groupe constitué par un radical phényle éventuellement substitué [par un radical alcoyle, trifluorométhyle, dialcoylaminométhyle, alcoyloxy, alcoylthio, dialcoylamino ou par un atome d'halogène tel que le fluor ou le chlore], un radical hétérocyclyle à 5 ou 6 chaînons contenant 1 seul hétéroatome tel que pyridyle-2 ou -3, thiényle-2 ou -3 furyle-2 ou -3 éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons ou un radical hétérocyclyle à 5 ou 6 chaînons contenant un atome d'oxygène ou de soufre et

le symbole $R_3$ représente un radical de formule générale:

$$R_4SO_2O— \qquad (V)$$

dans laquelle $R_4$ représente un radical alcoyle, trihalogénométhyle, ou phényle éventuellement substitué par un atome d'halogène ou par un ou plusieurs radicaux alcoyle ou nitro, étant entendu que

lorsque n = 0, le produit se présente sous forme bicyclooctène-2 ou -3 lorsque n = 1, le produit se présente sous forme bicyclooctène-2 et que les portions ou radicaux alcoyles sont (sauf mention spéciale) droits ou ramifiés et contiennent 1 à 4 atomes de carbone, caractérisé en ce que A — on fait agir une forme activée d'un acide $R_4SO_3H$, de formule générale:

$$(R_4SO_2)_2O \qquad (VIa)$$

$$\text{ou } R_4SO_2Hal \qquad (VIb)$$

dans laquelle $R_4$ est défini comme précédemment et Hal représente un atome d'halogène, sur une cétone de formule générale:

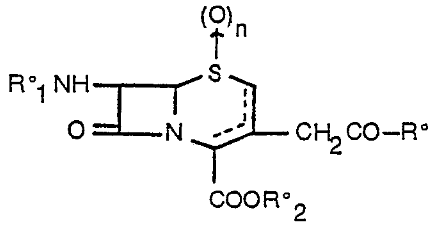

(VII)

[dans laquelle $R°_1$ est défini comme $R°_{1a}$ en a) ou en c), à l'exception pour $R_6$ de représenter un atome d'hydrogène lorsque $R°_1$ répond à la définition donnée en a) $R°_2$ est défini comme $R°_{2a}$ et n sont définis comme précédemment, et qui se présente sous forme bicyclooctène-2 ou -3 lorsque n = 0 et sous forme bicyclooctène-2 lorsque n = 1], ou sur un mélange de ses isomères, puis on réduit éventuellement le sulfoxyde obtenu lorsque n = 1, ou inversement on oxyde le produit obtenu en son S-oxyde, on élimine éventuellement les radicaux protecteurs, et sépare éventuellement les isomères obtenus, puis B — pour la préparation d'un produit pour lequel $R°_{1a}$ est défini comme en a), on acyle une céphalosporine obtenue selon A— pour laquelle $R°_{1a}$ représente un atome d'hydrogène, ou un mélange de ses isomères, au moyen d'un acide de formule générale:

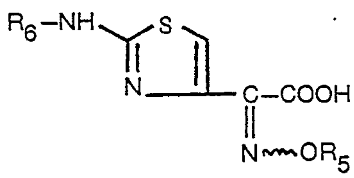

(X)

[dans laquelle $R_5$ et $R_6$ sont définis comme en a) à l'exception pour $R_6$ et pour $R^c_5$ (dans $R_5$) de représenter un atome d'hydrogène] ou d'un dérivé réactif de cet acide, puis on réduit éventuellement le sulfoxyde obtenu, ou inversement on transforme le produit obtenu en son S-oxyde, on élimine éventuellement les groupements protecteurs et sépare éventuellement les isomères obtenus.

**Claims for the Contracting States: BE CH DE FR GB IT LI LU NL SE**

1. A new cephalosporin derivative of the general formula:

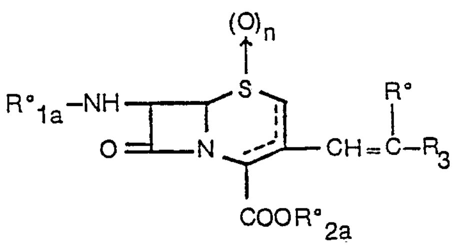

(I)

in which:

the symbol $R°_{1a}$ represents:

a) a radical of the general formula:

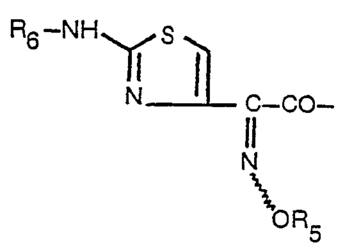

(II)

[[in which $R_5$ is a hydrogen atom, an alkyl, vinyl or cyanomethyl radical, an oxime-protecting group or a radical of the general formula:

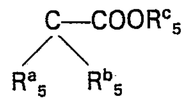

(III)

(in which $R^a_5$ and $R^b_5$, which are identical or different, are hydrogen atoms or alkyl radicals or together form an alkylene radical containing 2 or 3 carbon atoms, and $R^c_5$ is a hydrogen atom or an acid-protecting radical) and $R_6$ is a hydrogen atom or an amino-protecting radical]],

b) a hydrogen atom or

c) a benzhydryl radical, a trityl radical, an acyl radical of the general formula:

$$R_7CO— \qquad (IVa)$$

[[in which $R_7$ is an alkyl radical substituted by a phenyl or phenoxy radical]], a radical of the general formula:

$$R_8OCO— \qquad (IVb)$$

[[in which $R_8$ is an unsubstituted branched alkyl radical or a linear or branched alkyl radical carrying one or more substituents [chosen from amongst phenyl and phenyl substituted by an alkoxy, nitro or phenyl radical] or a vinyl radical]] or a radical of the general formula:

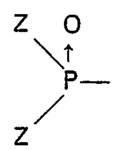

(IVc)

in which the symbols Z represent phenyl radical or radicals —OZ′, in which Z′ represents an alkyl, 2,2,2-trichloroethyl, phenyl or benzyl radical (it being possible for these last 2 radicals to be substituted by a halogen atom or an alkyl, alkoxy or nitro radical), or alternatively the symbols Z′ of the 2 substituents Z together form an alkylene radical containing 2 or 3 carbon atoms, or alternatively $R°_{1a}NH$ is replaced by a methyleneamino radical in which the methylene radical is substituted by a dialkylamino or aryl group (itself optionally substituted by 1 or more methoxy or nitro radicals),

the symbol $R°_{2a}$ represents a hydrogen atom or an acid-protecting radical,

the symbol R° represents a radical from a first group comprising a phenyl radical optionally substituted by an alkyl, trifluoromethyl, dialkylaminomethyl, alkoxy, alkylthio or dialkylamino radical or by a halogen atom such as fluorine or chlorine, a 5-membered or 6-membered heterocyclic radical containing only 1 heteroatom, such as pyridin-2-yl or pyridin-3-yl, thien-2-yl or thien-3-yl or furan-2-yl or furan-3-yl, optionally substituted by an alkyl, alkoxy or dimethylaminomethyl radical, or alternatively a radical from a second group comprising an alkyl radical containing 1 to 5 carbon atoms, a benzyl radical optionally substituted by a halogen atom or an alkyl, alkoxy, alkylthio, dialkylamino or trifluoromethyl radical, a methyl radical substituted by a 5-membered or 6-membered aromatic heterocycle such as pyridin-2-yl or pyridin-3-yl, thien-2-yl or thien-3-yl or furan-2-yl or furan-3-yl, a 3-membered to 6-membered cycloalkyl radical or a 5-membered or 6-membered heterocyclic radical containing an oxygen or sulphur atom, and

the symbol $R_3$ represents a radical of the general formula:

$$R_4SO_2O— \qquad (V)$$

in which $R_4$ represents an alkyl radical, a trihalogenomethyl radical or a phenyl radical optionally substituted by a halogen atom or by one or more alkyl or nitro radicals, it being understood that if $n = 0$, the product is in the form of a bicyclooct-2-ene or bicyclooct-3-ene, and if $n = 1$, the product is in the form of a bicyclooct-2-ene, and that the alkyl portions or radicals are linear or branched (unless otherwise mentioned) and contain 1 to 4 carbon atoms.

2. A cephalosporin derivative according to the Claim 1, characterised in that $R°_{1a}$ represents a radical of the general formula:

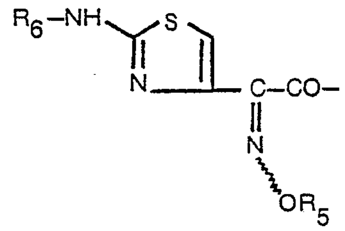

(II)

in which $R_6$ is defined as in Claim 1 and $R_5$ is a hydrogen atom, an alkyl, vinyl, cyanomethyhl, trityl, tetrahydropyranyl or 2-methoxyprop-2-yl radical or a radical of the general formula:

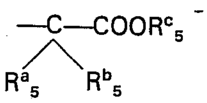

(III)

in which $R^a_5$ and $R^b_5$ are defined as in Claim 1 and $R^c_5$ is a hydrogen atom or a methoxymethyl, t-butyl, benzhydryl, benzyl, nitrobenzyl or p-methoxybenzyl radical.

3. A cephalosporin derivative according to Claim 1, characterised in that $R^o_{2a}$ is a hydrogen atom or a protecting radical chosen from amongst methoxymethyl, t-butyl, benzhydryl, benzyl, nitrobenzyl or p-methoxymethyl.

4. A cephalosporin derivative according to Claim 1, characterised in that n = 0 or 1, the symbol $R^o_{1a}$ is a hydrogen atom or a radical of the general formula $R_8OCO$— in which $R_8$ is defined as in Claim 1, the symbol $R^o_{2a}$ is a protecting radical, the symbol $R^o$ is an alkyl radical and the symbol $R_3$ is a radical of the general formula $R_4SO_2O$— in which $R_4$ is an alkyl radical, a trihalogenomethyl radical or a phenyl radical optionally substituted by an alkyl radical.

5. A cephalosporin derivative according to Claim 1, characterised in that n = 0 or 1, $R^o_{1a}$ is a hydrogen atom or a radical of the general formula $R_8OCO$— in which $R^8$ is a branched alkyl radical, $R^o_{2a}$ is a benzhydryl radical, $R^o$ is a methyl radical and $R_3$ is a radical of the general formula $R_4SO_2O$— in which $R_4$ is alkyl, trifluoromethyl or phenyl substituted by a methyl radical.

6. A process for the preparation of a product according to Claim 1 in which $R^o_{1a}$ is defined as in Claim 1 under a) or c) and $R^o_{2a}$, $R_3$ and n are defined as in Claim 1, characterised in that an activated form of an acid $R_4SO_3H$, of the general formula:

$$(R_4SO_2)_2O \qquad \text{(VIa)}$$

$$\text{or } R_4SO_2Hal \qquad \text{(VIb)}$$

in which $R_4$ is defined as in Claim 1 and Hal represents a halogen atom, is reacted with a ketone of the general formula:

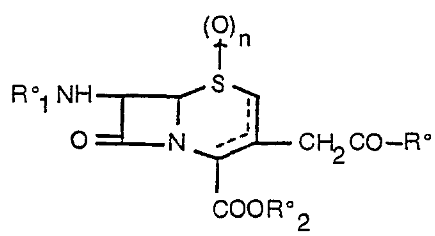

(VII)

[in which $R^o_1$ is defined in the same way as $R^o_{1a}$ above, except that $R_6$ cannot represent a hydrogen atom if $R^o_1$ corresponds to the definition given under a) in Claim 1, $R^o_2$ is defined in the same way as $R^o_{2a}$, and $R^o$ and n are defined as in Claim 1, and which is in the form of a bicyclooct-2-ene or bicyclooct-3-ene if n = 0 and in the form of a bicyclooct-2-ene if n = 1] or with a mixture of its isomers, the sulphoxide obtained is then reduced, if appropriate, if n = 1, the protecting radicals are removed, if appropriate, and the isomers obtained are separated, if appropriate.

7. A process for the preparation of a product according to Claim 1 in which $R^o_{1a}$ is a hydrogen atom and $R^o_{2a}$, $R_3$ and n are defined as in Claim 1, characterised in that the radical $R^o_{1a}$ is removed or the protecting radicals $R^o_{1a}$ and $R^o_{2a}$ are removed simultanteously from a product according to Claim 1 in which $R^o_{1a}$ is defined as in Claim 1 under c) and $R^o_{2a}$, $R_3$ and n are defined as in Claim 1, and the isomers obtained are then separated, if appropriate.

8. A process for the preparation of a product according to Claim 1 in which $R^o_{1a}$ is defined as in Claim 1 under a) and $R^o_{2a}$, $R_3$ and n are defined as in Claim 1, characterised in that a cephalosporin according to Claim 1 in which $R^o_{1a}$ is a hydrogen atom, or a mixture if its isomers, is acylated by means of an acid of the general formula:

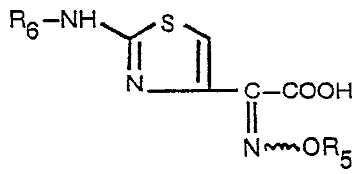

(X)

[in which $R_5$ and $R_6$ are defined as in Claim 1 under a), except that $R_6$ and $R^c_5$ (in $R_5$) cannot represent a hydrogen atom] or by means of a reactive derivative of this acid, the sulphoxide obtained is then reduced, if appropriate, the protecting groups are removed, if appropriate, and the isomers obtained are separated, if appropriate.

9. A process for the preparation of a product according to Claim 1 in which n equals 1, characterised in that a product according to Claim 1 in which $R^o_{1a}$ is defined as above and n equals 0 is oxidised and the isomers obtained are then separated, if appropriate.

**Claims for the Contracting State: AT**

Process for the preparation of a new cephalosporin derivative of the general formula:

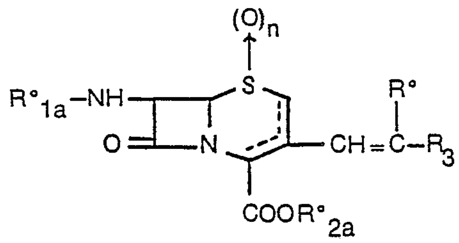

(I)

in which:

the symbol $R^o_{1a}$ represents:

a) a radical of the general formula:

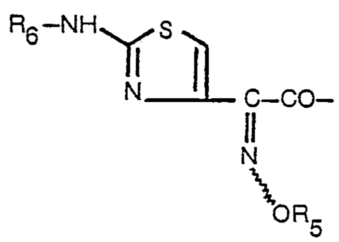

(II)

[[in which $R_5$ is a hydrogen atom, an alkyl, vinyl or cyanomethyl radical, an oxime-protecting group or a radical of the general formula:

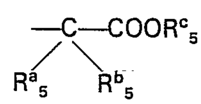

(III)

(in which $R^a_5$ and $R^b_5$, which are identical or different, are hydrogen atoms or alkyl radicals or together form an alkylene radical containing 2 or 3 carbon atoms, and $R^c_5$ is a hydrogen atom or an acid-protecting radical) and $R_6$ is a hydrogen atom or an amino-protecting radical]],

b) a hydrogen atom or

c) a benzhydryl radical, a trityl radical, an acyl radical of the general formula:

$$R_7CO— \quad \text{(IVa)}$$

[[in which $R_7$ is an alkyl radical substituted by a phenyl or phenoxy radical]], a radical of the general formula:

$$R_8OCO— \quad \text{(IVb)}$$

[[in which $R_8$ is an unsubstituted branched alkyl radical or a linear or branched alkyl radical carrying one or more substituents [chosen from amongst phenyl and phenyl substituted by an alkoxy, nitro or phenyl radical] or a vinyl radical]] or a radical of the general formula:

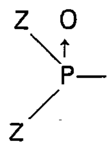

(IVc)

in which the symbols Z represent phenyl radicals or radicals —OZ′, in which Z′ represents an alkyl, 2,2,2-trichloroethyl, phenyl or benzyl radical (it being possible for these last 2 radicals to be substituted by a halogen atom or an alkyl, alkoxy or nitro radical), or alternatively the symbols Z′ of the 2 substituents Z together form an alkylene radical containing 2 or 3 carbon atoms, or alternatively $R^{o}_{1a}NH$ is replaced by a methyleneamino radical in which the methylene radical is substituted by a dialkylamino or aryl group (itself optionally substituted by 1 or more methoxy or nitro radicals),

the symbol $R^{o}_{2a}$ represents a hydrogen atom or an acid-protecting radical,

the symbol $R^{o}$ represents a radical from a first group comprising a phenyl radical optionally substituted by an alkyl, trifluoromethyl, dialkylaminomethyl, alkoxy, alkylthio or dialkylamino radical or by a halogen atom such as fluorine or chlorine, a 5-membered or 6-membered heterocyclic radical containing only 1 heteroatom, such as pyridin-2-yl or pyridin-3-yl, thien-2-yl or thien-3-yl or furan-2-yl or furan-3-yl, optionally substituted by an alkyl, alkoxy or dimethylaminomethyl radical, or alternatively a radical from a second group comprising an alkyl radical containing 1 to 5 carbon atoms, a benzyl radical optionally substituted by a halogen atom or an alkyl, alkoxy, alkylthio, dialkylamino or trifluoromethyl radical, a methyl radical substituted by a 5-membered or 6-membered aromatic heterocycle such as pyridin-2-yl or pyridin-3-yl, thien-2-yl or thien-3-yl or furan-2-yl or furan-3-yl, a 3-membered to 6-membered cycloalkyl radical or a 5-membered or 6-membered heterocyclic radical containing an oxygen or sulphur atom, and

the symbol $R_3$ represents a radical of the general formula:

$$R_4SO_2O— \qquad (V)$$

in which $R_4$ represents an alkyl radical, a trihalogenomethyl radical or a phenyl radical optionally substituted by a halogen atom or by one or more alkyl or nitro radicals, it being understood that if $n = 0$, the product is in the form of a bicyclooct-2-ene or bicyclooct-3-ene, and if $n = 1$, the product is in the form of a bicyclooct-2-ene, and that the alkyl portions or radicals are linear or branched (unless otherwise mentioned) and contain 1 to 4 carbon atoms, characterised in that:

A — an activated form of an acid $R_4SO_3H$, of the general formula:

$$(R_4SO_2)_2O \qquad (VIa)$$

$$\text{or } R_4SO_2Hal \qquad (VIb)$$

in which $R_4$ is defined as above and Hal represents a halogen atom, is reacted with a ketone of the general formula:

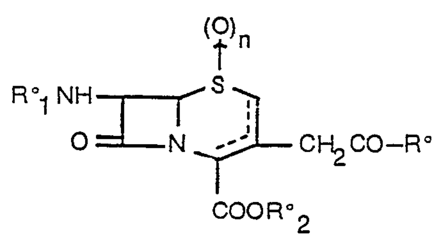

(VII)

[in which $R^{o}_1$ is defined in the same way as $R^{o}_{1a}$ under a) or under c), except that $R_6$ cannot represent a hydrogen atom if $R^{o}_1$ corresponds to the definition given under a), $R^{o}_2$ is defined in the same way as $R^{o}_{2a}$, and $R^{o}$ and n are defined as above, and which is in the form of a bicyclooct-2-ene or bicyclooct-3-ene if $n = 0$ and in the form of a bicyclooct-2-ene if $n = 1$] or with a mixture of its isomers, the sulphoxide obtained is then reduced, if appropriate, if $n = 1$, or, conversely, the product obtained is oxidised to its S-oxide, the protecting radicals are removed, if appropriate, and the isomers obtained are separated, if appropriate, and then

B — for the preparation of a product in which $R^{o}_{1a}$ is defined as under a), a cephalosporin obtained according to A — in which $R^{o}_{1a}$ represents a hydrogen atom, or a mixture of its isomers, is acylated by means of an acid of the general formula:

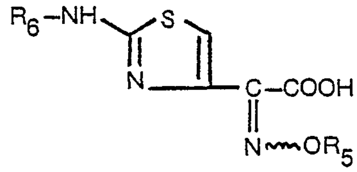

(X)

[in which $R_5$ and $R_6$ are defined as under a), except that $R_6$ and $R^c{}_5$ (in $R_5$) cannot represent a hydrogen atom] or by means of a reactive derivative of this acid, the sulphoxide obtained is then reduced, if appropriate, or, conversely, the product obtained is converted to its S-oxide, the protecting groups are removed, if appropriate, and the isomers obtained are separated, if appropriate.

**Patentansprüche für die Vertragsstaaten: BE CH DE FR GB IT LI LU NL SE**

1. Ein neues Derivat des Cephalosporins der allgemeinen Formel (I)

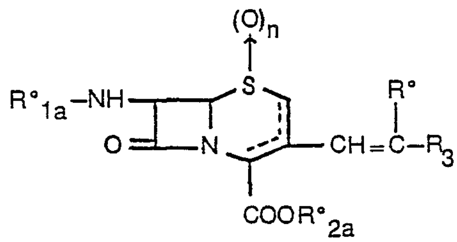

(I)

worin

das Symbol $R°_{1a}$ bedeutet:

a) einen Rest der allgemeinen Formel (II)

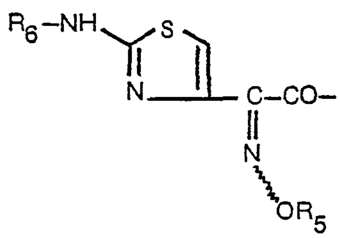

(II)

[[worin $R_5$ ein Wasserstoffatom, ein Alkyl-, Vinyl- oder Cyanomethylrest, eine Oximschutzgruppe oder ein Rest der allgemeinen Formel (III)

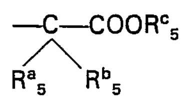

(III)

ist (worin $R^a{}_5$ und $R^b{}_5$, welche identisch oder verschieden sind, Wasserstoffatome, Alkylreste sind oder zusammen einen Alkylenrest mit 2 oder 3 Kohlenstoffatomen bilden und $R^c{}_5$ ein Wasserstoffatom oder ein Säureschutzrest ist) und $R_6$ ein Wasserstoffatom oder ein Aminoschutzrest ist]] oder

b) ein Wasserstoffatom oder

c) einen Benzylhydrylrest, Tritylrest, einen Acylrest der allgemeinen Formel (IVa)

$$R_7CO— \quad \text{(IVa)}$$

[[worin $R_7$ ein Alkylrest, substituiert durch einen Phenyl-oder Phenoxyrest ist]], einen Rest der allgemeinen Formel (IVb)

$$R_8OCO— \quad \text{(IVb)}$$

[[worin $R_8$ ein verzweigter, nicht substituierter Alkylrest oder ein gerader oder verzweigter Alkylrest mit einem oder mehreren Substituenten [ausgewählt unter Phenyl und substituiertem Phenyl (durch einen Alkyloxy-, Nitro- oder Phenylrest)] oder Vinyl ist]], einen Rest der allgemeinen Formel (IVc)

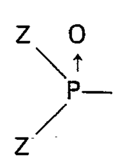

(IVc)

worin die Symbole Z Phenylreste oder —OZ' bedeuten, worin Z' einen Alkyl-, 2,2,2-Trichloräthyl-, Phenyl- oder Benzylrest bedeutet (wobei diese beiden letzteren Reste durch ein Halogenatom oder einen Alkyl-, Alkyloxy- oder Nitrorest substituiert sein können), oder auch die Symbole Z' von 2 Substituenten Z bilden zusammen einen Alkylenrest mit 2 oder 3 Kohlenstoffatomen, oder auch $R^{o}_{1a}NH$ ist durch einen Methylenaminorest ersetzt, worin der Methylenrest durch eine Dialkylaminogruppe oder Arylgruppe (welche ihrerseits gegebenenfalls durch ein oder mehrere Methoxy- oder Nitroreste substituiert ist) substituiert ist,

das Symbol $R^{o}_{2a}$ ein Wasserstoffatom oder einen Säureschutzrest bedeutet

das Symbol $R^{o}$ einen Rest einer ersten Gruppe bedeutet, gebildet durch einen gegebenenfalls substituierten Phenylrest [durch einen Alkyl-, Trifluormethyl-, Dialkylaminomethyl-, Alkyloxy-, Alkylthio-, Dialkylaminorest oder durch ein Halogenatom, wie Fluor oder Chlor], einen heterocyclischen Rest mit 5 oder 6 Ringgliedern, enthaltend ein einziges Heteroatom, wie Pyridyl-2 oder -3, Thienyl-2 oder -3 oder Furyl-2 oder -3, gegebenenfalls substituiert durch einen Alkyl-, Alkyloxy- oder Dimethylaminomethylrest oder auch einen Rest einer zweiten Gruppe, gebildet durch einen Alkylrest mit 1 bis 5 Kohlenstoffatomen, Benzyl, gegebenenfalls substituiert (durch ein Halogenatom oder einen Alkyl-, Alkyloxy-, Alkylthio-, Dialkylamino- oder Trifluormethylrest), einen Methylrest, substituiert durch einen aromatischen Heterocyclus mit 5 oder 6 Ringgliedern, wie Pyridyl-2 oder -3, Thienyl-2 oder -3 oder Furyl-2 oder -3, einen Cycloalkylrest mit 3 bis 6 Ringgliedern oder einen heterocyclischen Rest mit 5 oder 6 Ringgliedern, enthaltend ein Sauerstoff- oder Schwefelatom und

das Symbol $R_3$ einen Rest der allgemeinen Formel (V)

$$R_4SO_2O— \qquad (V)$$

bedeutet, worin $R_4$ einen Alkyl-, Trihalogenmethyl-, oder Phenylrest, gegebenenfalls substituiert durch ein Halogenatom oder durch einen oder mehrere Alkyl- oder Nitroreste, bedeutet, wobei, falls $n = 0$, das Produkt in Bicycloocten-2 oder -3-Form vorliegt und falls $n = 1$, das Produkt in Bicycloocten-2-Form vorliegt, und daß die Alkylteile oder -reste (falls nichts anderes angegeben ist) gerade oder verzweigt sind und 1 bis 4 Kohlenstoffatome enthalten.

2. Ein Derivat des Cephalosporins gemäß Anspruch 1, dadurch gekennzeichnet, daß $R^{o}_{1a}$ einen Rest der allgemeinen Formel (II)

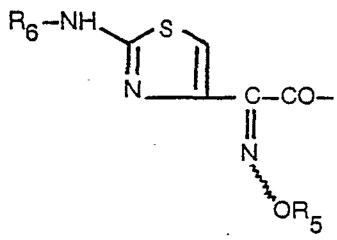

(II)

bedeutet, worin $R_6$ wie in Anspruch 1 definiert ist und $R_5$ ein Wasserstoffatom, ein Alkyl-, Vinyl-, Cyanomethyl-, Trityl-, Tetrahydropyranyl- oder 2-Methoxypropyl-2-Rest oder ein Rest der allgemeinen Formel (III)

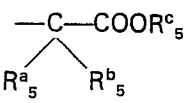

(III)

ist, worin $R^{a}_{5}$ und $R^{b}_{5}$ wie in Anspruch 1 definiert sind und $R^{c}_{5}$ ein Wasserstoffatom oder ein Methoxymethyl-, tert.Butyl-, Benzhydryl-, Benzyl-, Nitrobenzyl- oder p-Methoxybenzylrest ist.

3. Ein Derivat des Cephalosporins gemäß Anspruch 1, dadurch gekennzeichnet, daß $R^{o}_{2a}$ ein Wasserstoffatom oder ein Schutzrest, ausgewählt unter Methoxymethyl, tert.Butyl, Benzhydryl, Benzyl, Nitrobenzyl oder p-Methoxybenzyl, ist.

4. Ein Derivat des Cephalosporins gemäß Anspruch 1, dadurch gekennzeichnet, daß $n = 0$ oder 1,

das Symbol $R^{o}_{1a}$ ein Wasserstoffatom oder ein Rest der allgemeinen Formel $R_8OCO—$ ist, worin $R_8$ wie in Anspruch 1 definiert ist,

das Symbol $R^{o}_{2a}$ ein Schutzrest ist,

das Symbol $R^{o}$ ein Alkylrest ist und

das Symbol $R_3$ ein Rest der allgemeinen Formel $R_4SO_2O—$ ist, worin $R_4$ ein Alkyl-, Trihalogenmethyl- oder Phenylrest, gegebenenfalls substituiert durch einen Alkylrest, ist.

5. Ein Derivat des Cephalosporins gemäß Anspruch 1, dadurch gekennzeichnet, daß

$n = 0$ oder 1,

$R^{o}_{1a}$ ein Wasserstoffatom oder ein Rest der allgemeinen Formel

$R_8OCO—$ ist, worin $R_8$ ein verzweigter Alkylrest ist,

$R^{o}_{2a}$ ein Benzhydrylrest ist,

$R°$ ein Methylrest ist und

$R_3$ ein Rest der allgemeinen Formel $R_4SO_2O$— ist, worin $R_4$ Alkyl, Trifluormethyl oder Phenyl, substituiert durch einen Methylrest, ist.

6. Ein Verfahren zur Herstellung eines Produkts gemäß Anspruch 1, worin $R°_{1a}$ wie in Anspruch 1 in a) oder c) definiert ist und $R°_{2a}$, $R_3$ und n wie in Anspruch 1 definiert sind, dadurch gekennzeichnet, daß man eine aktivierte Form einer Säure $R_4SO_3H$ der allgemeinen Formel (VIa) oder (VIb)

$$(R_4SO_2)_2O \qquad (VIa)$$

$$\text{oder } R_4SO_2Hal \qquad (VIb)$$

worin $R_4$ wie in Anspruch 1 definiert ist und Hal ein Halogenatom bedeutet, auf ein Keton der allgemeinen Formel (VII)

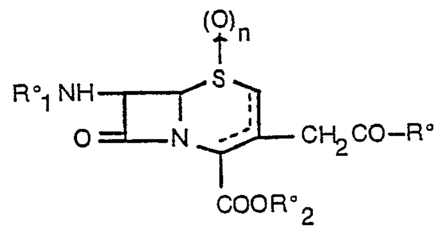

(VII)

[worin $R°_1$ wie $R°_{1a}$ vorstehend definiert ist, mit Ausnahme der Bedeutung eines Wasserstoffatoms für $R_6$, falls $R°_1$ der Definition in a) in Anspruch 1 entspricht, $R°_2$ wie $R°_{2a}$ definiert ist und $R°$ und n wie in Anspruch 1 definiert sind und das in Bicycloocten-2- oder -3-Form vorliegt, falls n = 0 und in Bicycloocten-2-Form, falls n = 1], oder auf ein Gemisch seiner Isomeren einwirken läßt, daß man dann gegebenenfalls das erhaltene Sulfoxid, falls n = 1, reduziert gegebenenfalls die Schutzreste entfernt und gegebenenfalls die erhaltenen Isomeren trennt.

7. Ein Verfahren zur Herstellung eines Produkts gemäß Anspruch 1, worin $R°_{1a}$ ein Wasserstoffatom ist und $R°_{2a}$, $R_3$ und n wie in Anspruch 1 definiert sind, dadurch gekennzeichnet, daß man den Rest $R°_{1a}$ entfernt oder gleichzeitig die Schutzreste $R°_{1a}$ und $R°_{2a}$ eines Produkts gemäß Anspruch 1 entfernt, worin $R°_{1a}$ wie in Anspruch 1 in c) definiert ist und $R°_{2a}$ eines Produkts gemäß Anspruch 1 entfernt, worin $R°_{1a}$ wie in Anspruch 1 in c) definiert ist und $R°_{2a}$, $R_3$ und n wie in Anspruch 1 definiert sind, und dann gegebenenfalls die erhaltenen Isomeren trennt.

8. Ein Verfahren zur Herstellung eines Produkts gemäß Anspruch 1, worin $R°_{1a}$ wie in Anspruch 1 in a) definiert ist und $R°_{2a}$, $R_3$ und n wie in Anspruch 1 definiert sind, dadurch gekennzeichnet, daß man ein Cephalosporin gemäß Anspruch 1, worin $R°_{1a}$ ein Wasserstoffatom ist oder ein Gemisch seiner Isomeren, mittels einer Säure der allgemeinen Formel (X)

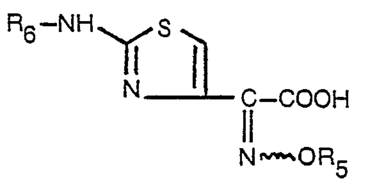

(X)

[worin $R_5$ und $R_6$ wie in Anspruch 1 in a) definiert sind, mit Ausnahme der Bedeutung eines Wasserstoffatoms für $R_6$ und für $R^c_5$ (in $R_5$)] oder eines reaktiven Derivats dieser Säure acyliert, dann gegebenenfalls das erhaltene Sulfoxid reduziert, gegebenenfalls die Schutzgruppen entfernt und gegebenenfalls die erhaltenen Isomeren trennt.

9. Ein Verfahren zur Herstellung eines Produkts gemäß Anspruch 1, worin n = 1, dadurch gekennzeichnet, daß man ein Produkt gemäß Anspruch 1, worin $R°_{1a}$ wie vorstehend definiert ist und n = 0 ist, oxydiert und dann gegebenefalls die erhaltenen Isomeren trennt.

**Patentanspruch für den Vertragsstaat: AT**

Verfahren zur Herstellung eines neuen Cephalosporinderivats der allgemeinen Formel

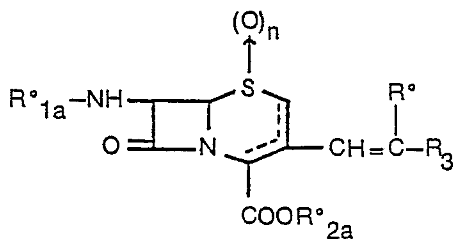

(I)

in welcher

das Symbol $R^{o}_{1a}$ bedeutet:

a) einen Rest der allgemeinen Formel:

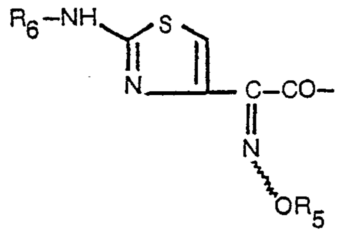

(II)

[[in welcher $R_5$ ein Wasserstoffatom, ein Alkyl-, Vinyl- oder Cyanomethylrest, eine Oximschutzgruppe oder ein Rest der allgemeinen Formel

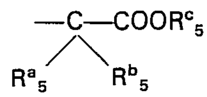

(III)

(worin $R^a_5$ und $R^b_5$, die gleich voneinander verschieden sind, Wasserstoffatome oder Alkylreste sind oder gemeinsam einen Alkylenrest mit 2 oder 3 Kohlenstoffatomen bilden und $R^c_5$ ein Wasserstoffatom oder eine Säureschutz gruppe ist) und $R_6$ ein Wasserstoffatom oder eine Aminoschutzgruppe ist]] oder

b) ein Wasserstoffatom oder

c) einen Benzhydryl oder Tritylrest, einen Acylrest der allgemeinen Formel:

$$R_7CO\text{—} \qquad \text{(IVa)}$$

[[worin $R_7$ ein Alkylrest, substituiert durch einen Phenyl- oder Phenoxyrest, ist]], einen Rest der allgemeinen Formel:

$$R_8OCO\text{—} \qquad \text{(IVb)}$$

[[worin $R_8$ ein nicht substituierter verzweigter Alkylrest oder ein geradkettiger oder verzeigter Alkylrest mit einem oder mehreren Substituenten [ausgewählt aus Phenyl und substituiertem Phenyl (durch einen Alkyloxy-, Nitro- oder Phenylrest)] oder Vinylrest ist]], einen Rest der allgemeinen Formel:

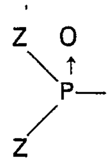

(IVc)

in welcher die Symbole Z Phenylreste oder —OZ' bedeuten, worin Z' einen Alkyl-, 2,2,2-Trichloräthyl-, Phenyl- oder Benzylrest (diese zwei letzten Reste können durch ein Halogenatom oder einen Alkyl-, Alkyloxy- oder Nitrorest substituiert sein) darstellt oder die Symbole Z' der beiden Substituenten Z gemeinsam einen Alkylenrest mit 2 oder 3 Kohlenstoffatomen bilden, oder $R^{o}_{1a}NH$ ersetzt ist durch einen Methylenaminorest, worin die Methylengruppe substituiert ist durch eine Dialkylamino- oder Arylgruppe (letztere ist selbst gegebenenfalls durch einen oder mehrere Methoxy- oder Nitroreste substituiert),

das Symbol $R^{o}_{2a}$ eine Wasserstoffatom oder eine Säureschutzgruppe darstellt,

das Symbol $R^{o}$ bedeutet: einen Rest aus einer ersten Gruppe, bestehend aus einem Phenylrest, gegebenenfalls substituiert (durch einen Alkyl-, Trifluormethyl-, Dialkylaminomethyl-, Alkyloxy-, Alkylthio-, Dialkylaminorest oder durch ein Halogenatom wie Fluor oder Chlor), einem Heterocyclylrest mit 5 oder 6 Gliedern, der ein einziges Heteroatom enthält, z.B. 2- oder 3-Pyridyl, 2- oder 3-Thienyl oder 2- oder 3-Furyl, gegebenenfalls substituiert durch einen Alkyl-, Alkyloxy- oder Dimethylaminomethylrest, oder einen Rest aus einer zweiten Gruppe, bestehend aus einem Alkylrest mit 1 bis 5 Kohlenstoffatomen, dem Benzylrest, gegebenenfalls substituiert (durch ein Halogenatom oder einen Alkyl-, Alkyloxy-, Alkylthio-, Dialkylamino, oder Trifluormethylrest), einem Methylrest, substituiert durch einen aromatischen Heterocyclus mit 5 oder 6 Gliedern, z.B. 2- oder 3-Pyridyl, 2- oder 3-Thienyl- oder 2- oder 3-Furyl, einem Cycloalkylrest mit 3 bis 6 Gliedern oder einem Heterocyclylrest mit 5 oder 6 Gliedern, der ein Sauerstoff- oder Schwefelatom enthält, und

das Symbol $R_3$ bedeutet: einen Rest der allgemeinen Formel:

$$R_4SO_2O\text{—} \qquad \text{(V)}$$

in welcher $R_4$ einen Alkyl- oder Trihalogenmethylrest oder einen Phenylrest, gegebenenfalls substituiert durch ein Halogenatom oder durch eine oder mehrere Alkyl- oder Nitrogruppen, darstellt, wobei, wenn n = 0, die Verbindung in der 2- oder 3-Bicycloocten-Form vorliegt, und wenn n = 1, die Verbindung in der 2-Bicycloocten-Form vorliegt und die Alkylteile oder -reste (ohne gesonderte Erwähnung) geradkettig oder verzweigt sind und 1 bis 4 Kohlenstoffatome enthalten, dadurch gekennzeichnet, daß man

A — eine aktivierte Form einer Säure $R_4SO_3H$ der allgemeinen Formel:

$$(R_4SO_2)_2O \quad \text{(VIa)}$$

$$\text{oder } R_4SO_2Hal \quad \text{(VIb)}$$

worin $R_4$ die obige Bedeutung hat und Hal ein Halogenatom darstellt, auf ein Keton der allgemeinen Formel:

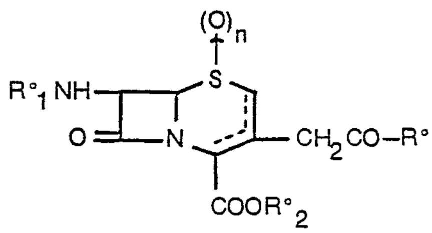

(VII)

[worin $R°_1$ die für $R°_{1a}$ unter a) oder c) angegebene Bedeutung hat, mit Ausnahme eines Wasserstoffatoms für $R_6$, wenn $R°_1$ der unter a) angegebenen Bedeutung entspricht; $R°_2$ die für $R°_{2a}$ angegebene Bedeutung hat und R° und n die zuvor angegebenen Bedeutungen haben, und welche in der 2- oder 3-Bicycloocten-Form vorliegt, wenn n = 0, und in der 2-Bicycloocten-Form, wenn n = 1] oder eine Mischung seiner Isomeren einwirken läßt, dann gegebenenfalls das erhaltene Sulfoxid, wenn n = 1, reduziert oder umgekehrt die erhaltene Verbindung zu ihrem S-Oxid oxidiert, gegebenenfalls die Schutzgruppen entfernt und gegebenenfalls die erhaltenen Isomeren trennt, dann

B — zur Herstellung einer Verbindung, worin $R°_{1a}$ die unter a) angegebene Bedeutung hat, ein nach A — erhaltenes Cephalosporin, worin $R°_{1a}$ ein Wasserstoffatom darstellt, oder eine Mischung seiner Isomeren mittels einer Säure der allgemeinen Formel

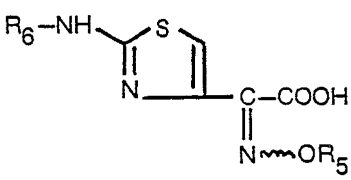

(X)

[worin $R_5$ und $R_6$ die unter a) angegebene Bedeutung haben, mit Ausnahme eines Wasserstoffatoms für $R_6$ und $R^c_5$ (in $R_5$)] oder eines reaktionsfähigen Derivats dieser Säure acyliert, dann gegebenenfalls das erhaltene Sulfoxid reduziert oder umgekehrt die erhaltene Verbindung in ihr S-Oxid überführt, gegebenenfalls die Schutzgruppen entfernt und gegebenenfalls die erhaltenen Isomeren trennt.